# EUROPEAN PATENT APPLICATION

(11) **EP 1 418 185 A1**
(43) Date of publication of application: **12.05.2004**
(21) Application number: 02025161.7
(22) Date of filing: 11.11.2002
(51) Int. Cl.: C07K 14/705

(54) **Use of EDG2 receptor in an animal model of heart failure**

(71) Applicant: Aventis Pharma Deutschland GmbH, 65929 Frankfurt am Main (DE)
(72) Inventor: Kostenis, Evi, Dr., 36323 Grebenau (DE); Wohlfart, Paulus, Dr., 64625 Bensheim (DE); Huber, Jochen, Dr., 67133 Maxdorf (DE); Rosport, Kai, DI., 81371 München (DE); Bueltmann, Andreas, Dr., 81543 München (DE); Baumgartner, Christine, Dr., 80336 München (DE); Muench, Goetz, Dr., 80799 München (DE); Ungerer, Martin, Dr., 80799 München (DE)

(57) **Abstract**

The invention refers to a transient transformed mammal which is useful as animal model for heart failure.

## Description

The invention refers to a transient transformed mammal which is useful as animal model for heart failure.

G protein-coupled receptors (GPCRs) play a central role in a multiplicity of physiological processes. It is assumed that in the human genome about 1000 genes code for this receptor family. Approximately 60 % of the pharmaceuticals presently available through prescription act as GPCR agonists or antagonists. This underlines the importance of this receptor class for the pharmaceutical research industry. Owing to the size and importance of said protein family and in view of the fact that physiological ligands are still unknown for many GPCRs (orphan GPCRs), it can be assumed that this receptor class will be one of the most important reservoirs for suitable target proteins in the search for novel medicinal substances in the future.

GPCRs are a family of integral membrane proteins which are located on cell surfaces. They receive signals from extracellular signaling substances (e.g. hormones, neurotransmitters, peptides, lipids) and transfer these signals into the cell interior via a family of guanine nucleotide-binding proteins, the "G proteins". Depending on the receptor specificity, the G protein activated and the cell type, these receptors induce various signal transduction pathways.
All GPCR polypeptide chains fold into seven α-helices which span across the phospholipid bilayer of the cell membrane. The seven membrane passages result in the formation of extra- and intracellular loops which allow extracellular ligand binding and intracellular coupling of G proteins. For this reason, GPCRs are also denoted seven-pass transmembrane receptors.

All G protein-coupled receptors act according to a common basic pattern: binding of an extracellular ligand leads to a conformational change in the receptor protein which enables the receptor protein to contact a G protein. G protein-mediated signal transduction cascades in the cell finally lead to a biological response of the cell.

G proteins are heterotrimeric proteins which consist of the subunits α, β and γ. They are located on the inside of the cell membrane via lipid anchors. Coupling of activated GPCRs to G proteins induces a GDP/GTP exchange at the Gα subunit and dissociation of the heterotrimeric G protein into an α and a βγ subunit. Both the activated α subunit and the βγ complex are able to interact with intracellular effector proteins.
Activation of membrane-bound adenylate cyclase (AC) by Gαs-type G proteins, for example, leads to an increase in the intracellular cAMP level or, in the case of activation by Gαi-type G proteins, to the decrease therein. Gq-type G proteins activate phospholipase C (PLC) which catalyzes the formation of inositol 1,4,5-triphosphate (IP3) and diacylglycerol (DAG). These molecules lead to the release of Ca²⁺ from intracellular storage organelles or to activation of proteinkinase C (PKC).

The polynucleotide sequence and the amino acid sequence of the human EDG2 (Endothelial Differentiation Gene 2) has been made available to the public. The sequence is available for example from NCBI (Accession: NM_001401). The protein sequence is available from Swiss Prot (Accession: Q 92633). Cloning of the receptor from a human lung cDNA library was published in "An et al., Biochem. Biophys. Res. Commun. 24, 231 (1997)".
The full length sequence encodes a 359 amino acid protein which belongs to the superfamily of guanine nucleotide-binding protein-coupled receptors (GPCR). Human EDG2 mRNA is widely distributed in human tissues with the highest abundance in brain. HEK293 cells expressing the human EDG2 protein showed an elevated response to lysophosphatidic acid (LPA) in a serum response element reporter gene assay, which was LPA concentration dependent and specific to LPA. The mouse counterpart of EDG2 protein was also identified as a receptor for LPA.

Lysophosphatidic acid (LPA) and sphingosine 1-phosphate (S1P) are potent phospholipid mediators with diverse biological activities. Their appearance and functional properties suggest possible roles in development, wound healing, and tissue regeneration. The growth-stimulating and other complex biological activities of LPA and S1P are attributable in part to the activation of multiple G protein-mediated intracellular signaling pathways. Several heterotrimeric G proteins, as well as Ras- and Rho-dependent pathways play central roles in the cellular responses to LPA and S1P.

Within the scope of this invention and in all cases used and without any exemption mammal shall not encompass the human species (Homosapiens) or an individual of Homosapiens of part of a body of a human.

The invention refers to a myocardial cell of a mammal which cell contains an adenoviral vector sequence for simultaneous expression of a G protein coupled receptor EDG2 and GFP (Green Fluorescent Protein).

The adenoviral vector sequence consists preferably of a recombinant E 1/E 3 deficient adenovirus which expresses the G protein coupled receptor EDG2 and GFP under control of two independent promoters. Such promoters could be two CMV promoters.

The myocardial cell of a mammal which contains an adenoviral vector sequence as aforementioned expresses the G protein coupled receptor EDG2 and GFP and contains therefore protein of the G protein coupled receptor EDG2 and protein of GFP.

The myocardial cell which contains an adenoviral vector sequence is preferably the cell of a rabbit, a mouse or a rat.

The invention refers also to production of a myocardial cell which cells contains an adenoviral vector sequence for simultaneous expression of G protein coupled receptor EDG2 and GFP wherein
a] the heart of a mammal is removed by state of the art veterinary medicine operative techniques,
b] the heart is perfused and digested with collagenase,
c] the isolated cardiomyocytes are infected with an adenoviral vector consisting of a recombinant E1/E3 deficient adenovirus which allows for expression of the G protein coupled receptor EDG2 and GFP under control of two independent promoters. Such promoters are preferably two CMV promoters.

Furthermore the invention refers to a mammal having a myocardium which contains an adenoviral vector for simultaneous expression of a G protein coupled receptor EDG2 and GFP. This adenoviral vector sequence of the mammals consists preferably of a recombinant E1/E3 deficient adenovirus which allows for expression of the G protein coupled receptor EDG2 and GFP under control of two independent promoters. Such two independent promoters are preferably two CMV promoters.

The invention refers also to a mammal having a myocardium which contains a protein of G protein coupled receptor EDG2 and a protein of GFP. Such a mammal having a myocardium with an adenoviral vector for simultaneous expression of a G protein coupled receptor EDG2 and GFP and/or having a myocardium with protein of G protein coupled receptor EDG2 and protein of GFP is preferably a rabbit, a mouse, or a rat. Furthermore the invention refers to production of a mammal having a myocardium with an adenoviral vector for simultaneous expression of a G protein coupled receptor EDG2 and GFP and/or having a myocardium with protein of G protein coupled receptor EDG2 and protein of GFP wherein
a] an adenoviral vector sequence for simultaneous expression of G protein coupled receptor EDG2 and GFP is provided,
b] a mammal is provided,
c] the adenoviral vector system from a] is transferred into the myocardium of the mammal from b] by means of a catheter.

The invention concerns also use of a mammal having a myocardium with an adenoviral vector for simultaneous expression of a G protein coupled receptor EDG2 and GFP and/or having a myocardium with protein of G coupled receptor EDG2 and protein of GFP for producing myocardial cells which can be taken for a method for identification of a compound which modifies the activity of G protein coupled receptor EDG2.

The invention refers to a method for identification of a compound which modifies the activity of receptor EDG2 wherein
a] a transformed cell from a heart muscle which expresses the receptor EDG2 or a fusion protein comprising the receptor EDG2 is provided,
b] possibly a treatment of the cell from a] is performed by use of isoproterenol and/or lyophosphatidic acid,
c] a chemical compound is provided,
d] the cell from a] or b] is brought in contact with the chemical compound from c],
e] the contractility of a cell from d] is determined and is brought in relation to the contractility of a cell which has the same characteristics as a cell from a] but which has not brought in contact with a chemical compound from c] and wherein a relative enhancement or reduction of contractility of the cell which has brought in contact with a chemical compound according to d] by this compound demonstrates the ability of such compound to modify the activity of receptor EDG2.

The invention refers furthermore to a method for identification of a compound which modifies the activity of receptor EDG2, wherein
a] a transformed cell from a heart muscle which expresses the receptor EDG2 or a fusion protein comprising the receptor EDG2 is provided,
b] possibly a treatment of the cell from a] is performed by use of proterenol and/or lysophosphatidic acid,
c] a chemical compound is provided,
d] the cell from a] or b] is brought in contact with the chemical compound from c],
e] the contractility of a cell from d] is determined and is brought in relation to contractility of a cell of same cell type as a cell according to a] but which does not express a receptor EDG2 or a fusion protein comprising a receptor EDG2 wherein a relative enhancement or reduction of contractility of the cell which expresses a receptor EDG2 or a fusion protein comprising a receptor EDG2 by a compound demonstrates the ability of such compound to modify the activity of receptor EDG2.

The invention refers furthermore to an adenoviral vector consisting of one polynucleotide of the following groups:
a] a polynucleotide having a sequence as specified in SEQ ID NO. 5,
b] a polynucleotide which is 95 % identical to the polynucleotide of SEQ ID NO. 5,
c] a polynucleotide which is at least of the same length as the polynucleotide of SEQ ID NO. 5 and which hybridizes to a polynucleotide of SEQ ID NO. 5 when applying highly stringent hybridization conditions.

The adenoviral vector sequence encompasses preferably a polynucleotide sequence which is encoding a protein of SEQ ID NO. 2.

Hybridization means assembly of two single polynucleotide strains which have complementary sequences to double stands. Hybridization might occur between two DNA-strand, one DNA- and one RNA-strand as well as between two RNA-strands. Forming of hybrid polynucleotide strands may start from a solution which contains double stranded polynucleotide molecules by heating this solution to separate the double strands in single stranded polynucleotides. The heating step could consist of boiling in a water bath during 10 to 20 minutes. When the solution is slowly cooled down to room temperature after it was heated the hybridization to double stranded molecules will occur. Under experimental conditions the hybridization is commonly carried out by means of hybridization filters which polynucleotides have been fixed upon by blotting or electrophoresis. Hybridization might be visualized by use of complementary polynucleotide molecules which carry a radioactive or fluorescenic label. Stringency describes the degree of correspondence under certain conditions. The demands with respect to correspondence are higher under high stringent conditions. Under circumstance of hybridization of nucleic acids the stringency conditions are adjusted in dependence of participating nucleic acids as well as use and objective. The conditions for a highly stringent hybridization are such that only very well fitting complementary molecules are able to hybridize. A very well fitting complementary polynucleotide exhibits for example a degree of identity of 90, 91, 92, 93, 94, 95, 96, 97, 98, 99 % with respect to the complementary partner molecule. Under low stringency hybridization occurs also between polynucleotide molecules which are only complementary within certain segments of the molecule or which have large sections with mismatched or unpaired base pairs.

A hybridization condition of high stringency could be a hybridization wherein the hybridization step in presence of a labeled probe will be carried out in an aqueous 2XSSC solution at 68°C during at least 2 hours, and the following washing steps consist of a first washing in 2XSSC/0,1 % SDS at room temperature for 5 minutes, a second washing in 1 XSSC/0,1 % SDS at 68°C for 1 hour, and a third washing in 0,2 % SSC/0,1 % SDS at 68°C for another hour.

A 2XSSC-, 1XSSC-, or 0,2 XSSC solution is obtained by dilution of a 20XSSC solution. A 20XSSC solution consists of 3 mol/I NaCl and 0,3 mol/l Na-Citrate. The skilled person is well known of other standard methods for hybridization of polynucleotides under stringent conditions. Advice is given to him in particular by textbooks as "Current Protocols in Molecular Biology (Wiley Interscience; ISBN: 0-471-50338-X; eds.: F.M. Ansubel, R. Brant, R.R. Kingston, D.J. Moore, J.G. Seidmann, K. Struhl).

The invention consists further of use of an adenoviral vector consisting of one polynucleotide of the following groups:
a] a polynucleotide having a sequence as specified in SEQ ID NO. 5,
b] a polynucleotide which is 95 % identical to the polynucleotide of SEQ ID NO. 5,
c] a polynucleotide which is at least of the same length as the polynucleotide of SEQ ID NO. 5 and which hybridizes to a polynucleotide of SEQ ID NO. 5 when applying highly stringent hybridization conditions
for constructing of transgenic mammals wherein the G protein coupled receptor EDG2 is transiently or permanently expressed in at least one tissue. Such a tissue is preferably a part of the heart of the mammal. Tissue could also consist of a part of brain, muscle, fat, liver, kidney or other organs of a mammal.

General technical aspects of the invention will be further explained within the following chapters

Adenoviruses can infect a wide variety of cell types and tissues in both dividing and non-dividing cells. This characteristic, together with their relative ease of preparation and purification, has led to their extensive use as gene vectors.
The virus can incorporate only about 2 kb of foreign DNA without significant affects on its stability or its infectivity. The introduction of longer sequences therefore requires the removal of some or all of the virus genes. There are a range of techniques for constructing recombinant adenoviruses.

Vectors can be utilized for (amongst other things): (i) cancer therapy to deliver genes that will lead to tumor suppression and elimination; (ii) gene therapy, i.e. to deliver genes to tissues to augment defective genes; (iii) supplementary therapy to deliver genes, expression of which will combat disease processes.

In the first generation of vectors, the E1 and/or E3 gene cassettes were removed, allowing the introduction of up to 6.5 kb of foreign DNA, often under the control of a heterologous promoter. In the case of the E1 deletions, care was taken to ensure the retention of the ITR and the packaging sequences. Removal of the E1 region had the additional apparent advantage of impairing the transcription of the E2 genes (which are E1 dependent) and consequently the replication of virus DNA and the production of the virus capsid proteins.
The defective E1 viruses could be propagated by infection of 293 cells, which provide the E1 gene products in trans. Although many of the initial studies in vitro provided much promise, it soon became evident that the expression of the transgene in vivo was only transient and was depressed because of the overwhelming immune response, mounted mainly against the virus capsid antigens as well as the expressed transgene. One of the reason for this was the observation that many cells harbored E1-like proteins that allowed the E2 genes to function, albeit at reduced levels. In turn, this facilitated virus DNA replication and the synthesis of the late structural antigens and the production of replication-competent adenovirus (RCA). It also became evident that, at higher m.o.i., the E1 dependence of E2 gene transcription could be ablated.

The next approach was to construct vectors (using suitable complementing cell lines) with some or all of the E2 genes excised and hence with the capacity to replicate virus DNA and to produce RCAs removed. Generation of RCAs could also be prevented by constructing cell lines that do not contain adenovirus sequences that overlap those in the vector. Nevertheless, the host immune response was still a major impediment to achieving persistent transgene expression and was particularly evident when repeated infections were attempted. A number of studies confirmed that the infecting recombinant virus itself was sufficient to induce the immune response, perhaps not surprising in view of the early activation of signaling cascades noted above and the potent antigenicity of the capsid components.

Other, rather more sophisticated vectors (third generation) have been constructed by deleting other virus genes (Amalfitano et al. 1988) and the latest of these have all or nearly all of the virus genes removed. These so-called 'gutless' vectors (Hardy et al., 1997) originally retained only the ITR and packaging sequences and required helper virus and appropriate complementing cells for propagation, followed by careful purification. Nevertheless, there were problems associated with these techniques, mainly due to contaminating helper virus and vector instability. A further development, which prevented the packaging of the helper virus, involved the use of the Cre-lox helper-dependent system.

The AdEasy™ system for the production of recombinant adenoviruses is commercially available from Qbiogene. The construction of a recombinant adenovirus is typically a two-step process in which the desired expression cassette is first assembled into a transfer vector, and subsequently transferred into the adenoviral genome by homologous recombination. Insertion of DNA by homologous recombination is the most efficient way of introducing a gene into an adenovirus vector for two reasons: 1) adenoviral DNAs are large, linear molecules that contain sites for almost all restriction enzymes and 2) the genome is too large (36 kb) to be easily manipulated.

With the AdEasy™ vector system, the backbone vector containing most of the adenoviral genome is used in super coiled plasmid form rather than as linear DNA.

The homologous recombination step is performed in Escherichia coli. In the AdEasy™ system the cDNA of interest is first cloned into a transfer vector. The resulting plasmid is then linearized with Pme I and co-transformed into E.coli strain BJ5183 together with pAdEasy-1, the viral DNA plasmid. The pAdEasy-1 is E1 and E3 deleted; its E1 functions can be complemented in 293A cells. Recombinations are selected with kanamycin and screened by restriction enzyme analysis. The recombinant adenoviral construct is then cleaved with Pac I to expose its ITR (Inverted Terminal Repeat) and transfected into QBI-293A cells to produce viral particles.

The homologous recombination step is mediated between a linearized transfer vector and an intact super coiled adenovirus plasmid. The kanamycin resistance gene present in the transfer vector allows for the selection of recombinants. Because the cleaved AdEasy™ transfer vectors yield only a low background of kanamycin-resistant colonies, the homologous recombination system has a high signal-to-noise ratio. The E. coli strain BJ5183 is not recA but is deficient in other enzymes that mediate recombination in bacteria and was selected for its higher efficiency of transformation and recombination capabilities. One recombination is achieved and verified, the adenoviral recombinant DNA can simply the transferred to a regular recA, endA strain such as DH5α for greater yields of DNA production. Due to its recA status, DH5α cannot be used to generate adenovirus recombinants by homologous recombination.

Green fluorescent protein (GFP) from Aequora victoria has rapidly become a standard reporter in many biological systems. GFP is unique among light-emitting proteins in that it does not require the presence of cofactors or substrates for the generation of light. In the jellyfish Aequora victoria GFP is acting in a calcium-dependent manner. When Ca⁺² binds another bioluminescent protein, aequorin, which transfers energy indirectly to GFP to trigger the release of green light. This energy transfer can be mimicked experimentally by exposure of GFP to standard long-wave ultraviolet light. There are GFP isoforms available which emit blue or red light and which are stable at elevated temperatures. GFP was first used to look into living cells by fluorescence microscopy to monitor protein localization and to visualize dynamic cellular events. A fusion between any cloned gene of interest and GFP can be produced by subcloning techniques and may be introduced into the organism of interest by transient or stable expression. The fate of the resulting protein inside the living cell can then be followed using conventional fluorescence microscopy. Detection does not require fixation or permenbilization of cells. Likewise a protein may be traced within an animals tissue by simultaneously expressing such protein and GFP.

Providing a cell includes its preparation, cultivation and further processing. Cells are provided, for example, by preparing suitable cell material from organs or tissues or by propagating suitable cell lines or microorganisms. Various suitable culture media can be used for cultivation. The cells are maintained at the optimum temperature for the organism. Where appropriate, preservatives, antibiotics, pH indicators, blood serum components, blood serum, auxiliaries or other substances are added to the growth medium used in each case. Processes for preparation, cultivation and further processing are described in standard textbooks (Example: Basic Cell Culture; Ed. J.M. Davis; IRL Press; 1994).

The application of recombinant techniques provides for a construct, which is to be expressed in a cell, to be present in the form of a polynucleotide sequence which can be prepared by a skilled worker in a routine manner with the aid of his specialist knowledge. The worker skilled in molecular biology / biochemistry can find the specialist knowledge for this, for example, in F.M. Ausubel et al.; Current Protocols in Molecular Biology; John Wiley & Sons; New York. A vector construct is prepared by incorporating a polynucleotide coding for the amino acid sequence of, for example, a GPCR into an expression vector. An expression vector is a vector in which a polynucleotide sequence can be expressed in a host cell into a protein. Vectors may be derived from plasmids, viruses or cosmids and must be capable of autonomous replication. They generally contain an origin of replication, cleavage sites for restriction enzymes and marker genes such as, for example, antibiotic resistance genes. In an expression vector, the polynucleotide sequence which is to be propagated or which has been introduced from the outside is under the functional control of a promoter. A promoter is a functional polynucleotide sequence of variable length, which is used to control transcription, i.e. synthesis of mRNA of a polynucleotide sequence immediately 3'- of said promoter. There are promoters which are active only in procaryotes, such as, for example, the lac, tac and trc promoters, and also promoters which are active only a eukaryotes, such as, for example, CMV or ADH promoters. In a preferred embodiment, the recombinant vector construct comprises an expression vector usable in eucaryotes and/or procaryotes. An expression vector contains a promoter which can be linked functionally to a polynucleotide sequence so that a protein encoded by said polynucleotide sequence is synthesized in an organism, for example a bacterium, fungus or the cell of a eucaryotic cell line. The promoter may be inducible, by means of tryptophan for example, or may be constitutively active. Examples of expression vectors are pUC18, pUC19, pBluescript, pcDNA3.1 etc.

Transfection is the introduction of foreign polynucleotide sequences into a host cell by means of a vector, and the subsequent propagation of said polynucleotide sequence to any number of identical copies.

A cell line is transiently transfected with a recombinant construct by means of routine methods which can be found by the skilled worker in the abovementioned Current Protocols in Molecular Biology, published by John Wiley & Sons, New York, or in Sambrook et al.; A Laboratory Manual, Cold Spring Harbor Laboratory, ISBN 0-87969-309-6. Examples of such routine methods are electroporation, Ca²⁺-phosphate coprecipitation and transfection by means of liposomes. The transfected genes may be expressed in the host cell by Western blotting of cell lysates of transfected cells in combination with an immunological detection method. For this too, the required laboratory protocols can be found by the skilled worker in the manuals mentioned above. Specific antibodies for immunodetection of GPCR receptors, which are suitable for carrying out the method of the invention, are commercially available.

A chemical compound is provided in particular by chemical synthesis or isolation of chemical substances from biological material.

The skilled worker may use routine methods for chemical synthesis of a compound or isolation of a substance from cells. Such methods are available to the skilled worker in textbooks such as Organic Synthesis Workbook; 1995; John Wiley & Sons; ISBN 3-527-30187-9, The Organic Chemistry of Drug Synthesis; 1998; John Wiley & Sons; ISBN 0-471-24510-0, or Bioactive Compounds from Natural Sources; 2001; Taylor & Francis; ISBN 0-7484-0890-8.

The compounds obtained by synthesis or isolation may be dissolved in a suitable solvent. Suitable solvents may contain water, buffer substances (e.g. Tris, HEPES, MOPS, etc.), monovalent and/or divalent ions (e.g. K⁺, Na⁺, Mg²⁺, Ca²⁺, etc.), acids (e.g. HCl, H₂SO₄, formic acid, acetic acid, etc.), bases (e.g. NaOH, etc.), alcohol (e.g. methanol, ethanol, glycerol), detergents (e.g. Na dodecyl sulfate, etc.), organic solvents (e.g. formamide, acetone, dimethyl sulfoxide, etc.) and other components, in particular solubilizers and stabilizers.

The skilled worker can contact the chemical compound with said cell line by using laboratory routine methods. Contacting may take place, for example, in Erlenmeyer vessels, tubes, Eppendorf vessels or on microtiter plates. Temperture-controlled incubators for which a constant temperature of, for example, 30°C or 37°C and fixed CO₂ or humidity conditions can be set may be used for said contacting. Contacting may in particular also be carried out in laboratory robot devices provided therefore (FLIPR). Contacting is possible for different periods of time, from a few seconds to minutes and up to several hours. The conditions to be chosen in each case depend on the receptor, the cell line and the chemical compound.

The final form of a pharmaceutical relates to the final formulation, for example, as tablet, granules, spray, solution, ointment, tincture or other formulation forms.

Processing to the final form refers to the preparation of the particular formulation in generally, the daily dose is in the range from 0.3 mg to 100 mg (typically from 3 mg to 50 mg) per day and per kilogram of body weight, for example, 3-10 mg/kg/day. An intravenous does may be, for example, in the range from 0.3 mg to 1.0 mg/kg and can most suitably be administered as in infusion of from 10 ng to 100 ng per kilogram and per minute. Suitable infusion solutions for these purposes may contain, for example,from 0.1 ng to 10 mg, typically from 1 ng to 10 mg, per milliliter. Single doses may contain, for example, from 1 mg to 10 g of the active substance. It is thus possible for ampoules for injections to contain, for example, from 1 mg to 100 mg, and for single-dose formulations which can be administered orally, such as, for example, tablets or capsules, to contain, for example, from 1.0 to 1000 mg, typically from 10 to 600 mg.

### Examples

The following examples disclose the invention without restricting it to the scope of these examples.

### Description of the experimental animals

The animals employed for the studies are ten-week-old female New Zealand white rabbits supplied by Asamhof, Kissing (Germany). At the start of the study the animals have a body weight of between 2.7 and 3.3 kg.

### Rearing and housing conditions of the experimental animals

The young are weaned at 30 days. To minimize weaning stress the dam is separated from the young so that the litter initially remains together. After about 10-14 days the young are housed in pairs in fattening cages (l x b x h = 28.5 x 60 x 34 cm) and then after about two weeks they are housed singly. The fatteners are introduced and removed in accordance with the "All in - All out" system to facilitate cleaning and disinfection.

The feed is produced in the in-house agricultural plant and is available to the rabbits ad libitum, as is drinking water.

Climate: The ventilation rate is 10,000 m³/h in summer and 3000 m³/h in winter. Particular attention is paid to avoiding drafts. In cold weather the temperature is maintained at about 15°C; overheating of the animal house in summer is prevented as far as possible. Young weaners are kept at about 19°C. Ammonia in the animal house is to be kept below 30 ppm, relative humidity below 70 %. The breeding house is illuminated for 16 hours at an intensity of about 20 lux.

### Study animal housing

The rabbits are brought in an air conditioned vehicle directly to the company, where they are allowed an adaptation period of 2 to 3 days to acclimatize themselves to the new diet and environment.

The rabbits are kept in conventional cages. The cage material consists of stainless steel with PVC inserts; the cage floor has an area of 4040 cm² and takes the form of a perforated bottom plate. The feces trays are cleaned daily and the cages are washed and hot-air-sterilized weekly. They are kept under constant conditions at a room temperature between 18 and 21°C and a relative humidity of 55 ± 5 %. As the animal house has windows the illumination corresponds to the natural night-day cycle with an intensity of at least 100 lux.

### Anesthesia and preparation for surgery

On the first day of the study, feed and water are available ad libitum to the rabbits until the start of anesthesia. Then the designated animal is weighed and undergoes a clinical examination, particularly of the cardiovascular system and the respiratory tract. The animals are provided with double intravenous access via indwelling catheters in the left and right lateral auricular veins (Venflon™ 08. x 25 mm), through which anesthesia is then induced with 1 % propofol (Disoprivan, Fresenius AG, Bad Homburg) in a dose of 7 mg/kg body weight i.v. Eye ointment (Vitamin A Dispersa, Ciba Vision®, Grossostheim) is applied to the cornea immediately after induction of anesthesia. After disappearance of the righting reflex the rabbits are shaved ventrally on the neck and on the chest between the elbows and the last rib and are then incubated by advancing a Magill tube with a cuff (internal diameter 2.5 to 3.5 mm, Rüsch AG, Waiblingen) into the trachea during inspiration.

To maintain anesthesia during the operation, the animals are given 2 % propofol (Disoprivan 2 %, Zeneca, Italy) i.v. in a dose of 12 to 14 ml/h via an infusion apparatus (Perfusor®, ED1-300, B. Braun, Melsungen AG). As analgesic the rabbits are given fentanyl (Fentanyl-Janssen 0.5 mg, Janssen-Cilag GmbH, Neuss) in a dose of 0.01 mg/kg i.v. immediately after intubation and then as required during the operation, roughly every 30 minutes, to maintain the surgical tolerance stage.

Using a small-animal ventilator (Anesthesia Workstation, Hallowell EMC, Völker GmbH, Kaltenkirchen), the rabbits are ventilated with 100 % oxygen at a breathing pressure of about 10 mm Hg, a respiratory volume of 8 to 12 ml/kg body weight and a respiratory rate of 29 to 32 breaths per minute, giving a CO₂ partial pressure of about 35 mm Hg in the expired air. Cardiovascular function is monitored intraoperatively via an ECG (Medtronic®, 9790 Programmer, Vitatron Medical B.V., Dieren, Netherlands). Respiration and circulation are monitored by pulse oximetry and capnometry.

### Aortic cross-clamping

Preparation of the right common carotid artery. Introduction of a polyurethane catheter (Cavafix®, 1.1* 1.7 mm/16G, ref. 4173589, B. Braun Melsungen AG, Melsungen, Germany) into the artery. After cannulation of the right common carotid artery the chest is opened through the third intercostal space. The virus is introduced into the myocardium using the Sigscreen® method, ensuring that the infused vector remains in that location in particular. The thorax is then closed with ligatures (Nylon®, 2-0 USP) and sutured (Vicryl® (3-0) and Nylon® (3-0)).

Analgesia: The animals receive carprofen (Rimadyl®, Pfizer, 4 mg/kg every 12 hours) and buprenorphine (Temgesic®, Boehringer, 0.01 mg/kg every 12 hours) for 72 hours after the operation.

### Euthanasia of the animals

On the last day of the study the animals undergo general anesthesia as described in Section 4. Euthanasia takes place in deep narcosis induced by pentobarbital 0.48 g/kg i.v. (Narcoren®, Rhone-Merieux GmbH, Laupheim).

### Postmortem macro pathological diagnosis and sampling

Immediately after death the heart is removed as quickly as possible by dividing the chest wall bilaterally at the level of the costal margin and completely disarticulating the sternum. The heart is separated from the afferent and efferent vessels and washed free of blood with cold sterile saline (isotonic sodium chloride solution, Delta-Pharm, Boehringer, Ingelheim) containing 5000 IU heparin. After gross pathological examination and weighing, the heart is preserved intact for further investigation. Hearts intended for single-cell isolation are briefly stored in a sterile tube of cold heparinized saline at about 4°C pending immediate processing. For determination of GFP fluorescence, frozen sections are prepared. Freshly removed hearts intended for cell microscopy are similarly washed with sterile saline, dried with cellulose, and then deep-frozen in a test tube of liquid nitrogen (-196°C) and stored at -80°C until further processing. The animals are autopsied in accordance with veterinary college guidelines, paying particular attention to evaluation of the extent of typical heart failure symptoms: ascites, pleural effusion, heart weight, heart shape, liver congestion and liver weight.

### Disposal of the carcasses

After collection in a deep-freeze cabinet at -20°C, the carcasses are fetched by the carcass disposal unit for disposal.

### Construction and Purification of Recombinant Adenovirus:

A human EDG2 receptor was cloned by using a PCR-based strategy on the basis of the coding sequence of the human EDG2 receptor. Recombinant (E1/E3-deficient) flag-tagged adenoviruses for this receptor (Ad-EDG2-GFP) were generated, expressing the transgene and green fluorescence protein (GFP) under control of two independent CMV promoters. As a control, Ad-GFP without further transgenes was used. Large virus stocks were prepared and adenoviral titers were determined using plaque titration and GFP expression titration in non E1-expressing cells.

### Cloning of EDG2

The DNA of the EDG2 receptor was amplified from cDNA from human brain by PCR using the forward primer 5'-gcggggggtaccaccatggctgccatctctacttccatcc-3' (SEQ ID NO. 5) and the reverse primer 5'-gcggggctcgagtcacttgtcgtcgtcgtccttatagtcaaccacagagtgatcattgct-3' (SEQ ID NO. 6).

The PCR reaction was performed at 58°C annealing for 1 min and 72°C amplification temperature for 1 min over 20 cycles with the Expand High Fidelity PCR System (Roche Molecular Biochemicals, Mannheim, Germany). Within the PCR reaction, a HA-tag Epitope of 9 amino acids from hemaglutinin of the human influenza A virus) was generated in-frame at the 3'-end of the gene.

The PCR fragment was cloned into the plasmid pAd-Shuttle (Q Biogene, Heidelberg, Germany) by using the restriction sites for Kpnl and Xhol and the sequence of resulting pAd Track-CMV-EDG2 was checked by sequencing (MediGenomix, Martinsried, Germany).

SEQ ID NO. 1 discloses the polynucleotide sequence of EDG2 comprising the coding region of a HA-tag within the 30 nucleotides of the 3'-end.

SEQ ID NO. 2 refers to the amino acid sequence of EDG2 comprising the 9 amino acid HA-tag of the C-terminus.

In SEQ ID NO. 8 the polynucleotide sequence of EDG2 having a 5'-HindIII and 3'Xhol site is disclosed. This EDG2 gene has been cloned into Hindlll/Xbal sites of pcDNA 3.1 (invitrogen). Such a vector construct is also suitable for amplifying the EDG2 gene.

Construction of recombinant flag-tagged adenovirus (pAD easy 1-EDG2-HA-GFP)

The plasmid pADTrack CMV-EDG2 c-HA was linearized with Pmel (New England Biolabs, Beverly, MA) overnight, dephosphorylated and purified (GFX DNA and Ge1 Purification Kit; Amersham Pharmacia Biotech, Uppsala, Sweden). For homologous recombination, electro competent E. coli BJ5183 (Stratagene, La Jolla, California) were cotransformed with 1 µg of the linearized plasmid pADTrack CMV EDG2 c-HA and 0.1 µg pAdeasyl at 2500 V, 200 W and 25 µFD (E. coli-pulser; Biorad, Heidelberg, Germany), plated and incubated overnight at 37°C. The resulting vector, pAdEasyl-edg2-cHA-GFP, contained the full recombinant adenoviral DNA for Transfection. The full DNA sequence is shown in SEQ ID NO. 5.

The colonies were checked after minipreparation of the plasmid DNA with Pacl and the positive clones were retransformed into E. coli DH5a.
For transfection (Effectene Transfection reagent; Qiagen, Hilden, Germany) of 293 cells, plasmid DNA was digested with Pacl. The cells were cultured for 7 days and harvested by scraping and centrifugation. The pellet was resuspended in Dulbecco's PBS and the cells were lysed by four repetitive freezing (-80°C) and thawing (37°C) cycles. Cell debris was removed by centrifugation and the lysate stored at -80°C. For plaque selection of recombinant virus, 293 cells were infected in Dulbecco's PBS for 1 hour at room temperature under gentle agitation with different serial dilutions of lysate from transfection. Following the infection, the cells were overlayed with growth medium containing 0.5 % agarose (1:1 mix of modified Eagles medium 2 x, Gibco Life technologies #21935, supplemented with 20 % Serum, 2x penicillin/streptomycin, 2x L-glutamin and agarose in water 1 %, Seacam). 5-14 days post infection the cell layer was monitored for formation of plaques which were picked using a pasteur pipette, resuspended in 0,5 ml Dulbeccos PBS and stored at -80°C. The plaques were used for further amplification rounds on 293 cells.

### Model of Heart Failure

New Zealand White rabbits were treated by rapid pacing at 360 beats/min after pacemaker implantation. Under this protocol, a tachycardia-induced heart failure (HF) develops reproducibly over two weeks. The average +dp/dtmax-value in failing hearts was 2200±320 mmHg/sec (vs. 3200±390 mmHg/sec in healthy controls; p<0.05), and LVEDP increased from 3.6±0.4 mmHg to 13±3.4 (p<0.05).

### Adenoviral Gene Transfer to Rabbit Myocardium

Before the start of rapid pacing, all rabbits received catheter-based adenoviral gene transfer (4x10¹⁰ pfu) to the myocardium. For the intervention, the rabbits were anesthetized with fentanyl and propofol. The efficacy of gene transfer was assessed in all hearts after the end of the experiments by investigating transverse freeze-cut sections for expression of GFP by fluorescence microscopy. Morphological changes were assessed after fixation with 4 % paraformaldehyde. Gene transfer led to reproducible transgene expression in ~50 % of cardiomyocytes.

### Shortening measurements in isolated cardiomyocytes

Contractility of infected cardiomyocytes was measured by an electro-optical monitoring system connected to online digitalized assessment of amplitude and velocity of shortening and of relaxation. Transgene-positive cardiomyocytes were identified by co-expression of GFP under fluorescent light. After the contraction amplitude reached stability, increasing concentrations of isoproterenol were applied at constant concentrations of lysophosphatidic acid (LPA; 10⁻⁵ mol/l); or increasing LPA concentrations were added to constant concentrations of isoproterenol (10⁻⁸ mol/l).

### Single cell contraction

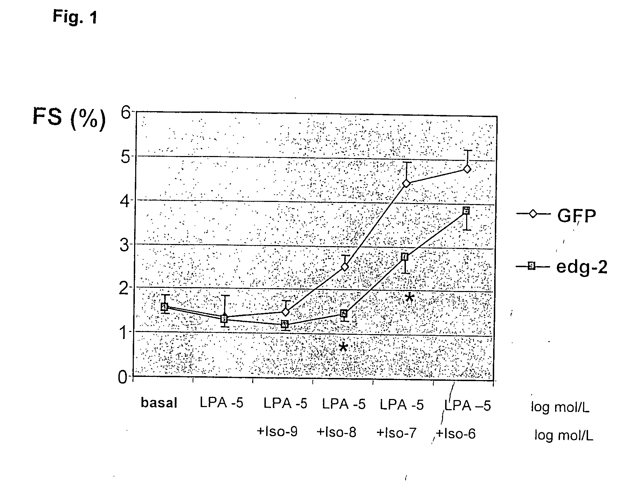

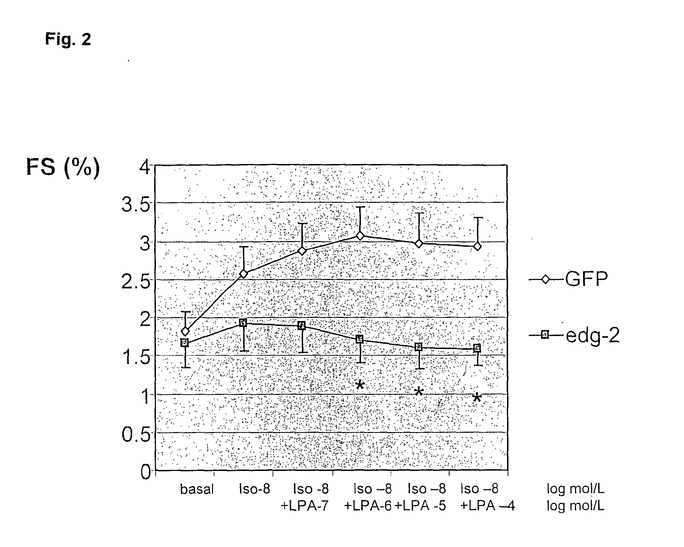

In order to investigate the effects of EDG2 on cardiomyocyte contractility, fractional shortening and velocity of shortening in single, isolated cardiomyocytes from failing hearts after ex vivo gene transfer was measured. At a concentration of lysophosphatidic acid (LPA) of 10⁻⁵ mol/l which does not alter basal contractility, increasing concentrations of isoproterenol had a significantly lower positively inotropic effect in EDG2-overexpressing cardiomyocytes (Fig. 1). After prestimulation with a low concentration of isoproterenol (10⁻⁸ mol/l), increasing concentrations of LPA showed a significant negatively inotropic effect in EDG2-overexpressing cardiomyocytes whereas no effect was observed in the control GFP group (Fig. 2). In the absence of prestimulation with isoproterenol, LPA has no effect on the contractility of cardiomyocytes.

### Western blot of infected cardiomyocytes

Cardiomyocytes were harvested 48 hours after adenoviral infection. The cells were homogenized and cytosolic extracts were then used for western blotting with antibodies against the HA tag or against EDG2. Horse radish peroxidase-coupled goat anti-rabbit antibodies by Dianova, Germany, were used as second antibodies.

### In vivo adenoviral delivery of transgene to failing heart

Overexpression of all transgenes was investigated by studying the co-expression of GFP in the hearts after in vivo gene transfer, since all transgenes were expressed together with GFP. A macroscopic slice of a rabbit heart infected with Ad-EDG 2-GFP showed GFP co-expression occurring throughout the left ventricle when determined by anti-GFP antibody staining.

### Transgene expression assessed by Western blotting

Western blotting documented the expression of EDG2 by means of an antibody directed against the HA tag or by a specific antibody against EDG2 in cardiomyocytes.

### Preparation and culture of adult ventricular cardiomyocytes and adenovirus infections

Single calcium-tolerant ventricular cardiomyocytes were isolated from failing White New Zealand rabbit hearts. Briefly, the hearts were perfused and digested with collagenase. The isolated cardiomyocytes were cultured in modified M199 on laminin-precoated dishes (5-10 µg/cm²) at a density of 1.5x10⁵ cells per cm² (at 5 % CO₂ and 37°C). For contraction experiments, the cells were infected with adenovirus (multiplicity of infection (moi) 1 pfu/cell) 5 hours after plating. 50-60 % of the infected cardiomyocytes expressed the transgene at this titer.

### Myocardial Contractility Measurement by Echocardiography and Intraventricular Tip Catheter

Left ventricular contractility was examined by echocardiography before the initiation of rapid pacing, after 1 week and after two weeks after the start of pacing. Tip catheter measurements were performed after 2 weeks of pacing. The rabbits were anesthetized; ECG was monitored continuously.

For echocardiography, a 7.5 MHz probe was fixed on a tripod. Standard sections were recorded, which were well reproducible. For tip catheter measurements, a Millar 3F tip catheter connected to a differentiating device was placed in the left ventricle via a sheath placed in the carotid artery. After definition of basal contractility and left ventricular pressure, 200 µL of NaCl (0.9 %) was injected as a negative control. Isoproterenol and lysophosphatidic acid (LPA) were infused intravenously at increasing doses. After a 20 min equilibration period, tip catheter measurements were carried out.

### Deterioration of LV dysfunction in pacing-induced heart failure

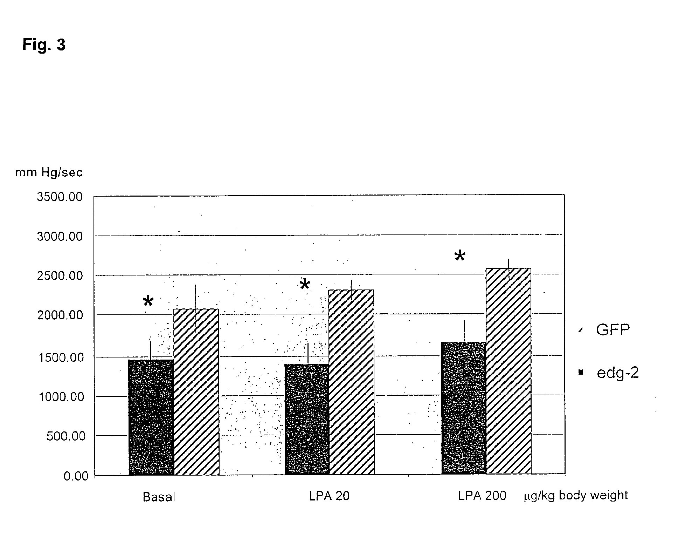

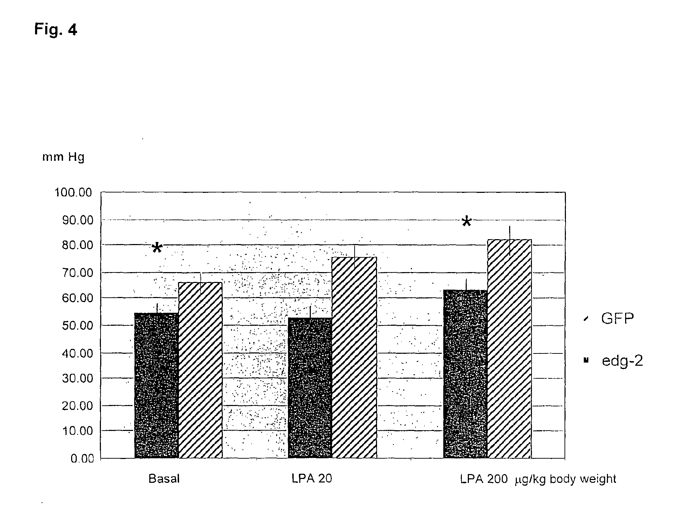

Fig. 3 shows tip catheterization measurements after 2 weeks of rapid pacing in rabbits suffering from severe heart failure (NYHA IV). In the EDG2-expression group, the first derivatives of LV pressure (dp/dt max) were significantly lower than in the Ad-GFP-infected control group at basal conditions and at increasing doses of LPA. This was also true for the increases in systolic LV pressure (Fig. 4).

Echocardiography showed a marked hypertrophy of the myocardium after 2 weeks in the EDG2-overexpressing hearts, which was also evidenced by decreases in systolic and diastolic diameters. The mean thicknesses of the posterior wall and of the septum of the LV were significantly greater in EDG2-overexpressing hearts compared to the GFP controls. The time course of LV fractional shortening (FS) was assessed by serial echocardiography during the two week-observation period. In both groups, FS declined gradually during the time period of rapid pacing.

### Description of Figures:

Fig. 1:
   Contraction amplitude of single cardiomyocytes isolated from failing hearts. The cardiomyocytes were infected ex vivo with either Ad-GFP or Ad-EDG2-GFP. Fractional shortening (FS) was determined in response to increasing concentrations of isoproterenol after prestimulation with 10 µM of LPA. Data represent means ± SEM.
Fig. 2:
   Contraction amplitude of single cardiomyocytes isolated from failing hearts. Similar to the experiments shown in Figure 1, FS was compared in cardiomyocytes after gene transfer with either Ad-GFP or Ad-EDG2-GFP in vitro. Fractional shortening was determined in response to increasing LPA concentrations after prestimulation with 10 µM of isoproterenol. Data represent means ± SEM.
Fig. 3:
   Maximum first derivative of left ventricular pressure (LV dp/dt max) at baseline and in response to increasing doses of LPA as determined by tip catheterization. In rabbits with terminal heart failure due to rapid pacing and after two weeks after gene transfer of either GFP or EDG2. Data represent means ± SEM. All measurements were done in 8 animals in triplicates *p<0.05 vs GFP.
Fig. 4:
   Left ventricular systolic pressure at baseline and in response to increasing doses of LPA as determined by tip catheterization in rabbits with terminal heart failure due to rapid pacing and after two weeks after gene transfer of either GFP or EDG2. Data represent means ± SEM. All measurements were done in 8 animals in triplicates. *p<0.05 vs GFP.

<110> Aventis Pharma Deutschland GmbH

<120> Use of EDG2 receptor in an animal model of heart
      failure

<130> DEAV2002/0083

<140>
<141>

<160> 8

<170>

<210> 1
<211> 1122
<212> DNA
<213> Homo sapiens

<400> 1
atggctgcca tctctacttc catccctgta atttcacagc cccagttcac agccatgaat 60
gaaccacagt gcttctacaa cgagtccatt gccttctttt ataaccgaag tggaaagcat 120
cttgccacag aatggaacac agtcagcaag ctggtgatgg gacttggaat cactgtttgt 180
atcttcatca tgttggccaa cctattggtc atggtggcaa tctatgtcaa ccgccgcttc 240
cattttccta tttattacct aatggctaat ctggctgctg cagacttctt tgctgggttg 300
gcctacttct atctcatgtt caacacagga cccaatactc ggagactgac tgttagcaca 360
tggctccttc gtcagggcct cattgacacc agcctgacgg catctgtggc caacttactg 420
gctattgcaa tcgagaggca cattacggtt ttccgcatgc agctccacac acggatgagc 480
aaccggcggg tagtggtggt cattgtggtc atctggacta tggccatcgt tatgggtgct 540
atacccagtg tgggctggaa ctgtatctgt gatattgaaa attgttccaa catggcaccc 600
ctctacagtg actcttactt agtcttctgg gccattttca acttggtgac ctttgtggta 660
atggtggttc tctatgctca catctttggc tatgttcgcc agaggactat gagaatgtct 720
cggcatagtt ctggaccccg gcggaatcgg gataccatga tgagtcttct gaagactgtg 780
gtcattgtgc ttggggcctt tatcatctgc tggactcctg gattggtttt gttacttcta 840
gacgtgtgct gtccacagtg cgacgtgctg gcctatgaga aattcttcct tctccttgct 900
gaattcaact ctgccatgaa ccccatcatt tactcctacc gcgacaaaga aatgagcgcc 960
acctttaggc agatcctctg ctgccagcgc agtgagaacc ccaccggccc cacagaaggc 1020
tcagaccgct cggcttcctc cctcaaccac accatcttgg ctggagttca cagcaatgat 1080
cactctgtgg tttatcccta tgacgtcccc gactatgcct ga                    1122

<210> 2
<211> 373
<212>
<213> Homo sapiens

<400> 2
maaistsipv isqpqftamn epqcfynesi affynrsgkh latewntvsk lvmglgitvc 60
ifimlanllv mvaiyvnrrf hfpiyylman laaadffagl ayfylmfntg pntrrltvst 120
wllrqglidt sltasvanll aiaierhitv frmqlhtrms nrrvvvvivv iwtmaivmga 180
ipsvgwncic diencsnmap lysdsylvfw aifnlvtfvv mvvlyahifg yvrqrtmrms 240
rhssgprrnr dtmmsllktv vivlgafiic wtpglvllll dvccpqcdvl ayekffllla 300
efnsamnpii ysyrdkemsa tfrqilccqr senptgpteg sdrsasslnh tilagvhsnd 360
hsvvypydvp dya 373

<210> 3
<211> 798
<212> DNA
<213> Aequorea victoria

<400> 3
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccctgaccta cggcgtgcag tgcttcagcc gctaccccga ccacatgaag 240
cagcacgact tcttcaagtc cgccatgccc gaaggctacg tccaggagcg caccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacaa cagccacaac gtctatatca tggccgacaa gcagaagaac 480
ggcatcaagg tgaacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgccgggatc actctcggca tggacgagct gtacaagtcc 720
ggactcagat ctcgagctca agcttcgaat tctgcagtcg acggtaccgc gggcccggga 780
tccaccggat ctagataa 798

<210> 4
<211> 265
<212>
<213> Aequorea victoria

<400> 4
mvskgeelft gvvpilveld gdvnghkfsv sgegegdaty gkltlkfict tgklpvpwpt 60
lvttltygvq cfsrypdhmk qhdffksamp egyvqertif fkddgnyktr aevkfegdtl 120
vnrielkgid fkedgnilgh kleynynshn vyimadkqkn gikvnfkirh niedgsvqla 180
dhyqqntpig dgpvllpdnh ylstqsalsk dpnekrdhmv llefvtaagi tlgmdelyks 240
glrsraqasn savdgtagpg stgsr 265

<210> 5
<211> 38306
<212> DNA

<213> adeno-associated virus 2

<220>
<223> Vector

<220>
<223> Adenoviral vector

<400> 5
attaannnnn atcatcaata atatacctta ttttggattg aagccaatat gataatgagg 60
gggtggagtt tgtgacgtgg cgcggggcgt gggaacgggg cgggtgacgt aggttttagg 120
gcggagtaac ttgtatgtgt tgggaattgt agttttctta aaatgggaag ttacgtaacg 180
tgggaaaacg gaagtgacga tttgaggaag ttgtgggttt tttggctttc gtttctgggc 240
gtaggttcgc gtgcggtttt ctgggtgttt tttgtggact ttaaccgtta cgtcattttt 300
tagtcctata tatactcgct ctgcacttgg ccctttttta cactgtgact gattgagctg 360
gtgccgtgtc gagtggtgtt tttttaatag gttttctttt ttactggtaa ggctgactgt 420
taggctgccg ctgtgaagcg ctgtatgttg ttctggagcg ggagggtgct attttgccta 480
ggcaggaggg tttttcaggt gtttatgtgt ttttctctcc tattaatttt gttatacctc 540
ctatgggggc tgtaatgttg tctctacgcc tgcgggtatg tattcccccg ggctatttcg 600
gtcgcttttt agcactgacc gatgaatcaa cctgatgtgt ttaccgagtc ttacattatg 660
actccggaca tgaccgagga gctgtcggtg gtgcttttta atcacggtga ccagtttttt 720
tacggtcacg ccggcatggc cgtagtccgt cttatgctta taagggttgt ttttcctgtt 780
gtaagacagg cttctaatgt ttaaatgttt ttttgttatt ttattttgtg tttatgcaga 840
aacccgcaga catgtttgag agaaaaatgg tgtctttttc tgtggtggtt ccggagctta 900
cctgccttta tctgcatgag catgactacg atgtgctttc ttttttgcgc gaggctttgc 960
ctgatttttt gagcagcacc ttgcatttta tatcgccgcc catgcaacaa agcttacatc 1020
ggggctacgc tggttagcat agctccgagt atgcgtgtca taatcagtgt gggttctttt 1080
gtcaaggttc ctggcgggga agtggccgcg ctggtccgtg cagacctgca cgattatgtt 1140
cagctggccc tgcgaaggga cctacgggat cgcggtattt ttgttaatgt tccgcttttg 1200
aatcttatac aggtctgtga ggaacctgaa tttttgcaat catgattcgc tgcttgaggc 1260
tgaaggtgga gggcgctctg gagcagattt ttacaatggc cggacttaat attcgggatt 1320
tgcttagaga tatattgaga aggtggcgag atgagaatta tttgggcatg gttgaaggtg 1380
ctggaatgtt tatagaggag attcaccctg aagggtttag cctttacgtc cacttggacg 1440
tgagggccgt ttgccttttg gaagccattg tgcaacatct tacaaatgcc attatctgtt 1500
ctttggctgt agagtttgac cacgccaccg gaggggagcg cgttcactta atagatcttc 1560
attttgaggt tttggataat cttttggaat aaaaaaaaaa acatggttct tccagctctt 1620
cccgctcctc ccgtgtgtga ctcgcagaac gaatgtgtag gttggctggg tgtggcttat 1680
tctgcggtgg tggatgttat cagggcagcg gcgcatgaag gagtttacat agaacccgaa 1740
gccagggggc gcctggatgc tttgagagag tggatatact acaactacta cacagagcga 1800
tctaagcggc gagaccggag acgcagatct gtttgtcacg cccgcacctg gttttgcttc 1860
aggaaatatg actacgtccg gcgttccatt tggcatgaca ctacgaccaa cacgatctcg 1920
gttgtctcgg cgcactccgt acagtaggga tcgtctacct ccttttgaga cagaaacccg 1980
cgctaccata ctggaggatc atccgctgct gcccgaatgt aacactttga caatgcacaa 2040
cgtgagttac gtgcgaggtc ttccctgcag tgtgggattt acgctgattc aggaatgggt 2100
tgttccctgg gatatggttc taacgcggga ggagcttgta atcctgagga agtgtatgca 2160
cgtgtgcctg tgttgtgcca acattgatat catgacgagc atgatgatcc atggttacga 2220
gtcctgggct ctccactgtc attgttccag tcccggttcc ctgcagtgta tagccggcgg 2280
gcaggttttg gccagctggt ttaggatggt ggtggatggc gccatgttta atcagaggtt 2340

tatatggtac cgggaggtgg tgaattacaa catgccaaaa gaggtaatgt ttatgtccag 2400
cgtgtttatg aggggtcgcc acttaatcta cctgcgcttg tggtatgatg gccacgtggg 2460
ttctgtggtc cccgccatga gctttggata cagcgccttg cactgtggga ttttgaacaa 2520
tattgtggtg ctgtgctgca gttactgtgc tgatttaagt gagatcaggg tgcgctgctg 2580
tgcccggagg acaaggcgcc ttatgctgcg ggcggtgcga atcatcgctg aggagaccac 2640
tgccatgttg tattcctgca ggacggagcg gcggcggcag cagtttattc gcgcgctgct 2700
gcagcaccac cgccctatcc tgatgcacga ttatgactct acccccatgt aggcgtggac 2760
ttctccttcg ccgcccgtta agcaaccgca agttggacag cagcctgtgg ctcagcagct 2820
ggacagcgac atgaacttaa gtgagctgcc cggggagttt attaatatca ctgatgagcg 2880
tttggctcga caggaaaccg tgtggaatat aacacctaag aatatgtctg ttacccatga 2940
tatgatgctt tttaaggcca gccggggaga aaggactgtg tactctgtgt gttgggaggg 3000
aggtggcagg ttgaatacta gggttctgtg agtttgatta aggtacggtg atctgtataa 3060
gctatgtggt ggtggggcta tactactgaa tgaaaaatga cttgaaattt tctgcaattg 3120
aaaaataaac acgttgaaac ataacacaaa cgattcttta ttcttgggca atgtatgaaa 3180
aagtgtaaga ggatgtggca aatatttcat taatgtagtt gtggccagac cagtcccatg 3240
aaaatgacat agagtatgca cttggagttg tgtctcctgt ttcctgtgta ccgtttagtg 3300
taatggttag tgttacaggt ttagttttgt ctccgtttaa gtaaacttga ctgacaatgt 3360
tacttttggc agttttaccg tgagattttg gataagctga taggttaggc ataaatccaa 3420
cagcgtttgt ataggctgtg ccttcagtaa gatctccatt tctaaagttc caatattctg 3480
ggtccaggaa ggaattgttt agtagcactc cattttcgtc aaatcttata ataagatgag 3540
cactttgaac tgttccagat attggagcca aactgccttt aacagccaaa actgaaactg 3600
tagcaagtat ttgactgcca cattttgtta agaccaaagt gagtttagca tctttctctg 3660
catttagtct acagttagga gatggagctg gtgtggtcca caaagttagc ttatcattat 3720
ttttgtttcc tactgtaatg gcacctgtgc tgtcaaaact aaggccagtt cctagtttag 3780
gaaccatagc cttgtttgaa tcaaattcta ggccatggcc aatttttgtt ttgaggggat 3840
ttgtgtttgg tgcattaggt gaaccaaatt caagcccatc tcctgcatta atggctatgg 3900
ctgtagcgtc aaacatcaac cccttggcag tgcttaggtt aacctcaagc tttttggaat 3960
tgtttgaagc tgtaaacaag taaaggcctt tgttgtagtt aatatccaag ttgtgggctg 4020
agtttataaa aagagggccc tgtcctagtc ttagatttag ttggttttga gcatcaaacg 4080
gataactaac atcaagtata aggcgtctgt tttgagaatc aatccttagt cctcctgcta 4140
cattaagttg catattgcct tgtgaatcaa aacccaaggc tccagtaact ttagtttgca 4200
aggaagtatt attaatagtc acacctggac cagttgctac ggtcaaagtg tttaggtcgt 4260
ctgttacatg caaaggagcc ccgtacttta gtcctagttt tccattttgt gtataaatgg 4320
gctctttcaa gtcaatgccc aagctaccag tggcagtagt tagagggggt gaggcagtga 4380
tagtaagggt actgctatcg gtggtggtga gggggcctga tgtttgcagg gctagctttc 4440
cttctgacac tgtgaggggt ccttgggtgg caatgctaag tttggagtcg tgcacggtta 4500
gcggggcctg tgattgcatg gtgagtgtgt tgcccgcgac cattagaggt gcggcggcag 4560
ccacagttag ggcttctgag gtaactgtga ggggtgcaga tatttccagg tttatgtttg 4620
acttggtttt tttgagaggt gggctcacag tggttacatt ttgggaggta aggttgccgg 4680
cctcgtccag agagaggccg ttgcccattt tgagcgcaag catgccattg gaggtaacta 4740
gaggttcgga taggcgcaaa gagagtaccc cagggggact ctcttgaaac ccattggggg 4800
atacaaaggg aggagtaaga aaaggcacag ttggaggacc ggtttccgtg tcatatggat 4860
acacggggtt gaaggtatct tcagacggtc ttgcgcgctt catctgcaac aacatgaaga 4920
tagtgggtgc ggatggacag gaacaggagg aaactgacat tccatttaga ttgtggagaa 4980
agtttgcagc caggaggaag ctgcaatacc agagctggga ggagggcaag gaggtgctgc 5040
tgaataaact ggacagaaat ttgctaactg attttaagta agtgatgctt tattattttt 5100
ttttattagt taaagggaat aagatccccg ggtactctag ttataactag aggatcttga 5160
tgtaatccaa ggttaggaca gttgcaaatc acagtgagaa cacagggtcc cctgtcccgc 5220

tcaactagca gggggcgctg ggtaaactcc cgaatcaggc tacgggcaag ctctccctgg 5280
gcggtaagcc ggacgccgtg cgccgggccc tcgatatgat cctcgggcaa ttcaaagtag 5340
caaaactcac cggagtcgcg ggcaaagcac ttgtggcggc gacagtggac caggtgtttc 5400
aggcgcagtt gctctgcctc tccacttaac attcagtcgt agccgtccgc cgagtccttt 5460
accgcgtcaa agttaggaat aaattgatcc ggatagtggc cgggaggtcc cgagaagggg 5520
ttaaagtaga ccgatggcac aaactcctca ataaattgca gagttccaat gcctccagag 5580
cgcggctcag aggacgaggt ctgcagagtt aggattgcct gacgaggcgt gaatgaagga 5640
cggccggcgc cgccgatctg aaatgtcccg tccggacgga gaccaagcga ggagctcacc 5700
gactcgtcgt tgagctgaat acctcgccct ctgattgtca ggtgagttat accctgcccg 5760
ggcgaccgca ccctgtgacg aaagccgccc gcaagctgcg cccctgagtt agtcatctga 5820
acttcggcct gggcgtctct gggaagtacc acagtggtgg gagcgggact ttcctggtac 5880
accagggcag cgggccaact acggggatta aggttattac gaggtgtggt ggtaatagcc 5940
gcctgttcca agagaattcg gtttcggtgg gcgcggattc cgttgacccg ggatatcatg 6000
tggggtcccg cgctcatgta gtttattcgg gttgagtagt cttgggcagc tccagccgca 6060
agtcccattt gtggctggta actccacatg tagggcgtgg gaatttcctt gctcataatg 6120
gcgctgacga caggtgctgg cgccgggtgt ggccgctgga gatgacgtag ttttcgcgct 6180
taaatttgag aaagggcgcg aaactagtcc ttaagagtca gcgcgcagta tttactgaag 6240
agagcctccg cgtcttccag cgtgcgccga agctgatctt cgcttttgtg atacaggcag 6300
ctgcgggtga gggatcgcag agacctgttt tttattttca gctcttgttc ttggcccctg 6360
ctctgttgaa atatagcata cagagtggga aaaatcctgt ttctaagctc gcgggtcgat 6420
acgggttcgt tgggcgccag acgcagcgct cctcctcctg ctgctgccgc cgctgtggat 6480
ttcttgggct ttgtcagagt cttgctatcc ggtcgccttt gcttctgtgt ggccgctgct 6540
gttgctgccg ctgccgctgc cgccggtgca gtatgggctg tagagatgac ggtagtaatg 6600
caggatgtta cgggggaagg ccacgccgtg atggtagaga agaaagcggc gggcgaagga 6660
gatgttgccc ccacagtctt gcaagcaagc aactatggcg ttcttgtgcc cgcgccatga 6720
gcggtagcct tggcgctgtt gttgctcttg ggctaacggc ggcggctgct tggacttacc 6780
ggccctggtt ccagtggtgt cccatctacg gttgggtcgg cgaacgggca gtgccggcgg 6840
cgcctgagga gcggaggttg tagccatgct ggaaccggtt gccgatttct ggggcgccgg 6900
cgaggggaat gcgaccgagg gtgacggtgt ttcgtctgac acctcttcga cctcggaagc 6960
ttcctcgtct aggctctccc agtcttccat catgtcctcc tcctcctcgt ccaaaacctc 7020
ctctgcctga ctgtcccagt attcctcctc gtccgtgggt ggcggcggca gctgcagctt 7080
ctttttgggt gccatcctgg gaagcaaggg cccgcggctg ctgctgatag ggctgcggcg 7140
gcggggggat tgggttgagc tcctcgccgg actgggggtc caagtaaacc ccccgtccct 7200
ttcgtagcag aaactcttgg cgggctttgt tgatggcttg caattggcca agaatgtggc 7260
cctgggtaat gacgcaggcg gtaagctccg catttggcgg gcgggattgg tcttcgtaga 7320
acctaatctc gtgggcgtgg tagtcctcag gtacaaattt gcgaaggtaa gccgacgtcc 7380
acagccccgg agtgagtttc aaccccggag ccgcggactt ttcgtcaggc gagggaccct 7440
gcagctcaaa ggtaccgata atttgacttt cgttaagcag ctgcgaattg caaaccaggg 7500
agcggtgcgg ggtgcatagg ttgcagcgac agtgacactc cagtagaccg tcaccgctca 7560
cgtcttccat tatgtcagag tggtaggcaa ggtagttggc tagctgcaga aggtagcagt 7620
ggccccaaag cggcggaggg cattcgcggt acttaatggg cacaaagtcg ctaggaagtg 7680
cacagcaggt ggcgggcaag attcctgagc gctctaggat aaagttccta aagttctgca 7740
acatgctttg actggtgaag tctggcagac cctgttgcag ggttttaagc aggcgttcgg 7800
ggaaaatgat gtccgccagg tgcgcggcca cggagcgctc gttgaaggcc gtccataggt 7860
ccttcaagtt ttgctttagc agtttctgca gctccttgag gttgcactcc tccaagcact 7920
gctgccaaac gcccatggcc gtctgccagg tgtagcatag aaataagtaa acgcagtcgc 7980
ggacgtagtc gcggcgcgcc tcgcccttga gcgtggaatg aagcacgttt tgcccaaggc 8040
ggttttcgtg caaaattcca aggtaggaga ccaggttgca gagctccacg ttggagatct 8100

tgcaggcctg gcgtacgtag ccctgtcgaa aggtgtagtg caatgtttcc tctagcttgc 8160
gctgcatctc cgggtcagca aagaaccgct gcatgcactc aagctccacg gtaacgagca 8220
ctgcggccat cattagtttg cgtcgctcct ccaagtcggc aggctcgcgc gtttgaagcc 8280
agcgcgctag ctgctcgtcg ccaactgcgg gtaggccctc ctctgtttgt tcttgcaaat 8340
ttgcatccct ctccaggggc tgcgcacggc gcacgatcag ctcactcatg actgtgctca 8400
tgaccttggg ggtaggttta agtgccgggt aggcaaagtg ggtgacctcg atgctgcgtt 8460
ttagtacggc taggcgcgcg ttgtcaccct cgagttccac caacactcca gagtgacttt 8520
cattttcgct gttttcctgt tgcagagcgt ttgccgcgcg cttctcgtcg cgtccaagac 8580
cctcaaagat ttttggcact tcgttgagcg aggcgatatc aggtatgaca gcgccctgcc 8640
gcaaggccag ctgcttgtcc gctcggctgc ggttggcacg gcaggatagg ggtatcttgc 8700
agttttggaa aaagatgtga taggtggcaa gcacctctgg cacggcaaat acggggtaga 8760
agttgaggcg cgggttgggc tcgcatgtgc cgttttcttg gcgtttgggg ggtacgcgcg 8820
gtgagaatag gtggcgttcg taggcaaggc tgacatccgc tatggcgagg ggcacatcgc 8880
tgcgctcttg caacgcgtcg cagataatgg cgcactggcg ctgcagatgc ttcaacagca 8940
cgtcgtctcc cacatctagg tagtcgccat gcctttcgtc cccccgcccg acttgttcct 9000
cgtttgcctc tgcgttgtcc tggtcttgct ttttatcctc tgttggtact gagcggtcct 9060
cgtcgtcttc gcttacaaaa cctgggtcct gctcgataat cacttcctcc tcctcaagcg 9120
ggggtgcctc gacggggaag gtggtaggcg cgttggcggc atcggtggag gcggtggtgg 9180
cgaactcaga gggggcggtt aggctgtcct tcttctcgac tgactccatg atctttttct 9240
gcctatagga gaaggaaatg gccagtcggg aagaggagca gcgcgaaacc acccccgagc 9300
gcggacgcgg tgcggcgcga cgtcccccaa ccatggagga cgtgtcgtcc ccgtccccgt 9360
cgccgccgcc tccccgggcg cccccaaaaa agcggatgag gcggcgtatc gagtccgagg 9420
acgaggaaga ctcatcacaa gacgcgctgg tgccgcgcac acccagcccg cggccatcga 9480
cctcggcggc ggatttggcc attgcgccca agaagaaaaa gaagcgccct tctcccaagc 9540
ccgagcgccc gccatcacca gaggtaatcg tggacagcga ggaagaaaga gaagatgtgg 9600
cgctacaaat ggtgggtttc agcaacccac cggtgctaat caagcatggc aaaggaggta 9660
agcgcacagt gcggcggctg aatgaagacg acccagtggc gcgtggtatg cggacgcaag 9720
aggaagagga agagcccagc gaagcggaaa gtgaaattac ggtgatgaac ccgctgagtg 9780
tgccgatcgt gtctgcgtgg gagaagggca tggaggctgc gcgcgcgctg atggacaagt 9840
accacgtgga taacgatcta aaggcgaact tcaaactact gcctgaccaa gtggaagctc 9900
tggcggccgt atgcaagacc tggctgaacg aggagcaccg cgggttgcag ctgaccttca 9960
ccagcaacaa gacctttgtg acgatgatgg ggcgattcct gcaggcgtac ctgcagtcgt 10020
ttgcagaggt gacctacaag catcacgagc ccacgggctg cgcgttgtgg ctgcaccgct 10080
gcgctgagat cgaaggcgag cttaagtgtc tacacggaag cattatgata aataaggagc 10140
acgtgattga aatggatgtg acgagcgaaa acgggcagcg cgcgctgaag gagcagtcta 10200
gcaaggccaa gatcgtgaag aaccggtggg gccgaaatgt ggtgcagatc tccaacaccg 10260
acgcaaggtg ctgcgtgcac gacgcggcct gtccggccaa tcagttttcc ggcaagtctt 10320
gcggcatgtt cttctctgaa ggcgcaaagg ctcaggtggc ttttaagcag atcaaggctt 10380
ttatgcaggc gctgtatcct aacgcccaga ccgggcacgg tcaccttttg atgccactac 10440
ggtgcgagtg caactcaaag cctgggcacg cgcccttttt gggaaggcag ctaccaaagt 10500
tgactccgtt cgccctgagc aacgcggagg acctggacgc ggatctgatc tccgacaaga 10560
gcgtgctggc cagcgtgcac cacccggcgc tgatagtgtt ccagtgctgc aaccctgtgt 10620
atcgcaactc gcgcgcgcag ggcggaggcc ccaactgcga cttcaagata tcggcgcccg 10680
acctgctaaa cgcgttggtg atggtgcgca gcctgtggag tgaaaacttc accgagctgc 10740
cgcggatggt tgtgcctgag tttaagtgga gcactaaaca ccagtatcgc aacgtgtccc 10800
tgccagtggc gcatagcgat gcgcggcaga acccctttga tttttaaacg gcgcagacgg 10860
caagggtggg ggtaaataat cacccgagag tgtacaaata aaagcatttg cctttattga 10920
aagtgtctct agtacattat ttttacatgt ttttcaagtg acaaaaagaa gtggcgctcc 10980

taatctgcgc actgtggctg cggaagtagg gcgagtggcg ctccaggaag ctgtagagct 11040
gttcctggtt gcgacgcagg gtgggctgta cctggggact gttgagcatg gagttgggta 11100
ccccggtaat aaggttcatg gtggggttgt gatccatggg agtttggggc cagttggcaa 11160
aggcgtggag aaacatgcag cagaatagtc cacaggcggc cgagttgggc ccctgtacgc 11220
tttgggtgga cttttccagc gttatacagc ggtcggggga agaagcaatg gcgctacggc 11280
gcaggagtga ctcgtactca aactggtaaa cctgcttgag tcgctggtca gaaaagccaa 11340
agggctcaaa gaggtagcat gcgggttcca ggcaaaggcc atccagtgta cgcccccagt 11400
ctcgcgaccg gccgtattga ctatggcgca ggcgagcttg tgtggagaaa caaagcctgg 11460
aaagcgcttg tcataggtgc ccaaaaaata tggcccacaa ccaagatctt tgacaatggc 11520
tttcagttcc tgctcactgg agcccatggc ggcagctgtt gttgatgttg cttgcttctt 11580
tatgttgtgg cgttgccggc cgagaagggc gtgcgcaggt acacggtttc gatgacgccg 11640
cggtgcggcc ggtgcacacg gaccacgtca aagacttcaa acaaaacata aagaagggtg 11700
ggctcgtcca tgggatccac ctcaaaagtc atgtctagcg cgtgggcgga gttggcgtag 11760
agaaggtttt ggcccaggtc tgtgagtgcg cccatggaca taaagttact ggagaatggg 11820
atgcgccaaa gggtgcgatc gcaaagaaac tttttctggg taatgctgtc aactgcggtc 11880
ttgcctataa gcggataggg gaagttagca gggtaggcct gtccttcgcg catggtgggg 11940
gcaaggtagc caacaaatcc agagttgttg tgttggtgta ggatgcccac ctgttggtag 12000
tccttgtatt tagtatcatc caccacctga cggctcatgg gctggaagtt tctaaagaag 12060
gagtacatgc ggtccttgta gctctctggg atatagaagc cctggtagcc aatgttgtag 12120
ttagctagca tttgtaccag gaaccagtct ttggtcatgt tacactgggc aacgttgtaa 12180
ccctccccgt caactgagcg cttaatttca aactcgttgg gggtaagcag gcggtcattg 12240
ccaggccagc tgacagaaga gtcaaaggta atggccacct tcttaaaggt gtggttgagg 12300
taaaaggttc catctaggta gggtatagag ccagagtagg tgtaataagg gtcgtagccc 12360
gagcccagtg atggggtttc cttagtctta aggcgcgtga aggcccagcc gcggaaagcc 12420
gcccagttgc gggaggggat ggatatgggc acgttggtag cgttggcggg tatagggtag 12480
agcatgttgg cggcggagag atagtcgtta aaggactggt cgttggtgtc gtttctaagc 12540
atggcctcaa gcgtggaggc ggtgttgtgg gccatgggga agaaggtggc gtaaaggcaa 12600
atgctatcaa acttaatgct ggctccgtca acccttaggt catttcctag ggagctctgc 12660
agaaccatgt taacatcctt cctgaagttc cactcgtagg tgtatgagcc cggcaggaga 12720
aggaggtttt taatggcaaa gaacttctga ggcacctgga tgtggaaggg cacatagcga 12780
ccattgccca gcaacattga gcggtagcgc aggccagcat tgcggtggtg gttaaatggg 12840
ttgacgttgt ccatatagtc aagggaccag cgtgctccaa ggttaatgta gcagtccact 12900
aacccgggag ccaccactcg cttgttcatg tagtcgtagg tgtttgggtt atcagaaatt 12960
tttacgttgg aaggactgta ctttagcttg tcgggcaaat acagcgctat gttggagtac 13020
aggaaatttc tccacaggtt ggcatttaga ttgatttcca tggcaaaatt atttccaact 13080
cttatttcat ttttatctga aaattctgta gcatcttttt cccatccatt ttcctgacct 13140
gttttaggtt ttaccttggt aagagtctct gtattaatca cacctcccag tggaaagcag 13200
taatttggaa gttcatcttc agttccatga ttttcaataa ttctaacatc tggatcatag 13260
ctgtcaacag cctgattcca catagaaaag tacctggttc tatcaccaat ggaatcaagc 13320
aaaagctggt atgaaagctc tgtgtttctg tcttgcaaat ctacaacagc attcaactgc 13380
gatgcttggc ccgccagaac acccatatta cccgtgctgt tgtaatacat tagaccaata 13440
aaattgtccc taaaagcaat gtaattaggc ctgttgggca tagattgttg gcccattagt 13500
tctcgtgagt taccttcctt aatagtgggc atgtaagaaa tatgagtgtc tggggtttct 13560
atatctacat cttcactgta caataccact ttaggagtca agttatcacc attgcctgcg 13620
gtcgcctcag tagttgagaa aaattgcatt tccacttgac tttctagctt tccattttgt 13680
tgctttacaa gaatgccttg ccctccattt tcatttgtgg gttttgcata tgaaccgtaa 13740
catggtttca ttggggtagt ctttttaagg actctcccag ctgcatgatt aatttcagtt 13800
tcgtaccact gagattctcc tatttgaggt tcaggttgaa atgttttatc ggcatattta 13860

ggtgtttgac cttcgacacc tatttgaata ccctcctttg taatatttat accagaataa 13920
ggcgcctgcc caaatacgtg agttttttgc tgctcagctt gctcgtctac ttcgtcttcg 13980
ttgtcatcgt cctcttcttc taggtttatt tcaagagcag tagcagcttc atcccattcg 14040
caaggatttg gggcaccctt gggagccagg gcgttgtagg cagtgccaga gtagggctta 14100
aaagtagggc ccctgtccag cacgccgcgg atgtcaaagt acgtggaagc catgtccagc 14160
acacggttat cacccacagc tagggtgaac cgcgccttgt acgagtacgc agtatcctca 14220
cggtccacag ggatgaaccg cagcgtcaaa cgctgggacc ggtctgtggt cacgtcgtgc 14280
gtaggcgcca ccgtggggtt tctaaacttg ttattcaggc tgaagtacgt ctcggtggcg 14340
cgggcaaact gcaccagccc ggggctcagg tactccgagg cgtcctggcc cgagatgtgc 14400
atgtaagacc actgcggcat catcgaaggg gtagccatct tggaaagcgg gcgcgcggcg 14460
gctcagcagc tcctctggcg gcgacatgga cgcatacatg acacacatac gacacgttag 14520
ctattcagaa gcatcgtcgg cgcttcaggg attgcacccc cagacccacg atgctgttca 14580
gtgtgctttg ccagttgcca ctggctacgg gccgcaacga tcgcggaccg ctggcggcgc 14640
ggcgcaggga cgcgcggcta ggacgggtta caacaacggc ggtcgggcct ggcagcacag 14700
gtttctgctg ggtgtcggcg gggggaggca ggtccagcgt tacgggtgtg tgctggccca 14760
gcactccggt agccatgggc gcgatgggac gggtggtggg caggccttgc tttagtgcct 14820
cctcgtacga gggaggctcg tctatttgcg tcaccagagt ttcttccctg tcggggcgcg 14880
gacgcttttc gccacgcccc tctggagaca ctgtctccac ggccggtgga ggctcctcta 14940
cgggagggcg gggatcaagc ttactgttaa tcttattttg cactgcctgg ttggccaggt 15000
ccaccacccc gctaatgcca gaggccaggc catctaccac cttttgttgg aaattttgct 15060
ctttcaactt atccctcagc atctggcctg tgctgctgtt ccaggccttg ctgccatagt 15120
tcttaacggt ggaaccgaaa tttttaatgc cgctccacag cgagccccag ctgaaggcgc 15180
caccgctcat attgctggtg ccgatatctt gccagtttcc catgaacggg cgcgagccgt 15240
gtcgcggggc cagagacgca aagttgatgt cttccattct acaaaatagt tacaggacca 15300
agcgagcgtg agagtccaga ctttttattt tgatttttcc acatgcaact tgtttttaat 15360
cagtgtctct gcgcctgcaa ggccacggat gcaattccgg gcacggcgcc aatcgccgcg 15420
gcgatcagtg gaataaggag gggcaggata ccgccgcgca tgcgacggtg cgacgcgcgc 15480
cgccgccggt ggtgcgcacg acgcatgccg cccgtcaggc cgtggccggc catgcccctc 15540
tacggtgcat tcttcctcgg aatcccggca ccgggaaacg gaggcggcag gtgagggcca 15600
tatctgcaag aaccacaaag accggctttt aaacgatgct ggggtggtag cgcgctgttg 15660
gcagcaccag ggtcctgcct ccttcgcgag ccaccctgcg cacggaaatc ggggccagca 15720
cgggctggcg acggcgacgg cggcggcggg ttccagtggt ggttcggcgt cgggtagttg 15780
ctcgtcttct ggggcggtag gtgtagccac gatagccggg ggtaggcgca atggaaggat 15840
gtagggcata ttcgggcagt agcgcgctgg cggcgccgta cttcctcgaa ccgcgcgggc 15900
gccggggggc tgaaacgcga aacatccacg ggtccgtttg cacctccgta gaggtcttgg 15960
acgcggccgc agcgaccgcc tgcaccgcgg catccgccac cgctgaggca accggggacg 16020
tttgtgtctc catgccctct gtggcggtgg caatactggt gctactggta gtgggtatct 16080
gaacgtccac ggtctgcacg cccagtcccg gcgccacctg cttgattggc cgcacgcgga 16140
cctcgggctc cagcccaggt tccacggtca ttttttccaa gacatcttcc agtcgctggc 16200
gcttgggtac catcagctgc acggtgggtg ccaagtcacc agactcgcgc tttaggccgc 16260
gcttttcttc ggacggtgca agcgcgggca gcacctgctg cagtgttacg ggctttaggc 16320
taggtgttgg gttgccctcg tccagcggca acgccagcat gtccttatgc cgctttccgt 16380
aggcaaactc cccgaggcgc tcgttggcct gctcaagcag gtcctcgtcg ccgtacacct 16440
catcatacac gcgcttgtag gtgcgggtgg agcgctcacc gggcgtaaag actacggtgg 16500
tgccgggtcg caaaacacgt tttacgcgtc gacctttcca ctgtacccgt cgcctgggcg 16560
cggtagcgtg cagcagttcc acctcgtcgt caagttcatc atcatcatct ttctttttct 16620
ttttgacccg ctttagcttt cggggcttgt aatcctgctc ttccttcttc ggggggccat 16680
agatctccgg cgcgatgacc tggagcatct cttctttgat tttgcgcttg gacatagctt 16740

cgttgcgcgc cgccgccgct ggatacatac aacagtacga gtctaagtag ttttttcttg 16800
caatctagtt gcgcgggggg cgggtgcgca cgggcacgcg caggccgcta accgagtcgc 16860
gcacccaata cacgttgccc ctgcgaccct gagtcatagc actaatggcc gcggctgctg 16920
cggcggccgc tcgtcgcctg gacctggggg gcacagtgac aatacccgcg gccagccttc 16980
gagcggcccg catggccgcc cgtcggccgg tgcgacgtgc gcggttaagc agggccgccg 17040
ccgcgcgttg ggcggcagtg ccgggtcggc ggcggtggcg acgtgctacg cgcctccgcc 17100
gtctcttcat tttagcatag cgccgggctc cgcgcaccac ggtctgaatg gccgcgtcca 17160
ctgtggacac tggtggcggc gtgggcgtgt agttgcgcgc ctcctccacc accgcgtcga 17220
tggcgtcatc gacggtggtg cgcccagtgc ggccgcgttt gtgcgcgccc cagggcgcgc 17280
ggtagtgccc gcgcacgcgc actgggtgtt ggtcggagcg cttcttggcc ccgccaaaca 17340
tcttgcttgg gaagcgcagg ccccagcctg tgttattgct gggcgatata aggatggaca 17400
tgcttgctca aaaagtgcgg ctcgatagga cgcgcggcga gactatgccc agggccttgt 17460
aaacgtaggg gcaggtgcgg cgtctggcgt cagtaatggt cactcgctgg actcctccga 17520
tgctgttgcg cagcggtagc gtcccgtgat ctgtgagagc aggaacgttt tcactgacgg 17580
tggtgatggt gggggctggc gggcgcgcca aaatctggtt ctcgggaaag cgattgaaca 17640
cgtgggtcag agaggtaaac tggcggatga gttgggagta gacggcctgg tcgttgtaga 17700
agctcttgga gtgcacgggc aacagctcgg cgcccaccac cggaaagttg ctgatctggc 17760
gcgtggagcg gaaggtcacg gggtcttgca tcatgtctgg caacgaccag tagacctgct 17820
ccgagccgca ggttacgtca ggagtgcaaa gcagggtcca tgagcggatt ccggtctgag 17880
ggtcgccgta gttgtatgca aggtaccagc tgcggtactg ggtgaaggtg ctgtcattgc 17940
ttattaggtt gtaactgcgt ttcttgctgt cctctgtcag gggtttgatc accggtttct 18000
tctgaggctt ctcgacctcg ggttgcgcag cgggggcggc agcttcggcc gctgcttcgg 18060
cctcagcgcg cttctcctca gcccgtgtgg caaaggtgtc gccgcgaatg gcatgatcgt 18120
tcatgtcctc caccggctgc attgccgcgg ctgccgcgtt ggagttctct tccgcgccgc 18180
tgccactgct gttgctgccg cctgcgccac ccccgccctg ttcggtgtca tctttcaagc 18240
tcgcctggta ggcgtccaca tccaacagtg cgggaatgtt accaccctcc agatcatcgt 18300
aggtgatcct aaagccctcc tggaagggtt gccgcttgcg gatgcccaac aagttgctca 18360
ggcggctgtg ggtgaagtcc accccgcatc ctggcagcaa aatgatgtct ggatggaagg 18420
cttcgtttgt atatacccca ggcatgacaa gaccagtgac ggggtcaaac cccagtctga 18480
agttgcgggt gtcaaacttt accccgatgt cgctttccag aaccccgttc tgtctgccca 18540
ctttcaagta gtgctccacg atcgcgttgt tcataaggtc tatggtcatg gtctcggagt 18600
agttgccctc gggcagcgtg aactccaccc actcgtattt cagctccacc tgattgtcct 18660
tagtaggcaa gcgcgacacc atcacccgcg ccttaaactt attggtaaac atgaactcgt 18720
tcacatttgg catgttggta tgcaggatgg ttttcaggtc gccgccccag tgcgaccggt 18780
cgtcaagatt gatggtctgt gtgcttgcct cccccgggct gtagtcattg ttttgaatga 18840
ccgtggtcag aaagttgctg tggtcgttct ggtagttcag ggatgccaca tccgttgact 18900
tgttgtccac caggtacaca cgggtggtgt cgaatagggg tgccaactca gagtaacgga 18960
tgctgtttct ccccccggta ggccgcaggt accgcggagg cacaaacggc gggtccaggg 19020
gagcatcgaa gggagaaccc agcgccgccg ccactggcgc cgcgctcacc acactctcgt 19080
aggagggagg aggaccttcc tcatacatcg ccgcgcgccg catactaagg ggaatacaag 19140
aaaaccaacg ctcggtgcca tggccttggt gagtttttta ttttgcatca tgcttttttt 19200
tttttaaaac attctcccca gcctggggcg aaggtgcgca aacgggttgc cactccctcc 19260
caaatccagg acgctgctgt cgtctgccga gtcatcgtcc tcccacacca gaccccgctg 19320
acggtcgtgc ctttgacgac gggtgggcgg gcgcgggcct ggcacgtccc tgtgctcctg 19380
cgcgtacgtc ttccatctac tcatcttgtc cactaggctc tctatcccgt tgttgggaaa 19440
tgccggaggc aggttttttt cgcgctgcgg ctgcagcagc gagttgttta ggtactcctc 19500
ctcgcccagc aggcgcgggc gggtggtgcg agtgctggta agagacccta tcaagcttgg 19560
aaatgggcta ctagcatctg accgcggggc cgcagcgcct agatcggaca agctgcttgg 19620

cctgcggaag ctttcctttc gcagcgccgc ctctgcctgc tcgcgctgtt gcaactctag 19680
cagggtctgc ggttgcgggg aaaacacgct gtcgtctatg tcgtcccaga ggaatccatc 19740
gttaccctcg ggcacctcga atcccccggt gtagaaacca gggggcggta gccagtgcgg 19800
gttcaagatg gcattggtga aatactcggg gttcacggcg gccgcgcgat gcaagtagtc 19860
cattaggcgg ttgataaacg gccggtttga ggcatacatg cccggttcca tgttgcgcgc 19920
ggtcatgtcc agcgccacgc tgggcgttac cccgtcgcgc atcaggttaa ggctcacgct 19980
ctgctgcacg tagcgcaaaa tgcgctcctc ctcgctgttt aaactgtgca acgaggggat 20040
cttctgccgc cggttggtca gcaggtagtt tagggttgcc tccaggctgc ccgtgtcctc 20100
ctgccccagc gcgcggctga cacttgtaat ctcctggaaa gtatgctcgt ccacatgcgc 20160
ctgacctatg gcctcgcggt acagtgtcag caagtgacct aggtatgtgt cccgggacac 20220
gctgccactg tccgtgaagg gcgctattag cagcagcaac aggcgcgagt tgggcgtcag 20280
caagctagac acggtcgcgc ggtcgcctgt gggagcccgc accccccaca gcccctgcaa 20340
gtttttgaaa gcctggctca ggtttacggt ctgcaggcct tgtctactgg tctggaaaaa 20400
atagtctggc ccagactggt acacctcact ttgcggtgtc tcagtcacca ttagccgcag 20460
tgcgctcaca aagttggtgt agtcctcctg tccccgcggc acgttggcgg gctgtgtact 20520
caggaaggcg tttagtgcaa ccatggagcc caggttgccc tgctgctgcg cgcgctcacg 20580
ctgcgccacg gcctcgcgca catcccccac cagccggtcc aggttggtct gcacgttgcc 20640
gctgttgtaa cgagccacgc gctgaagcag cgcgtcgtag accaggccgg cctcgtcggg 20700
ccggatggcc ctgttttcgg ccagcgcgtt tacgatcgcc agcaccttct cgtgcgtggg 20760
gtttgcgcgc gccgggacca ccgcttccag aattgcggag agccggttgg cctgcggctg 20820
ctgccggaac gcgtcaggat tgcgcgcagt cagcgacatg atgcggtcca tgacctggcg 20880
ccagtcgtcc gtggagttaa ggccggacgg ctggctctgc agcgccgccc gcaccgccgg 20940
gtccgttgcg tcttgcatca tctgatcaga aacatcaccg cttagtactc gccgtcctct 21000
ggctcgtact catcgtcctc gtcatattcc tccacgccgc cgacgttgcc agcgcgcgcg 21060
ggtgccaccg ccagcccagg tccggcccca gctgcctcca gggcgcgtcg gcttggggcc 21120
cagcgcaggt cagcgcccgc gtcaaagtag gactcggcct ctctatcgcc gctgcccgtg 21180
ccagccaggg ccctttgcag gctgtgcatc agctcgcggt cgctgagctc gcgccgccgg 21240
ctcacgctca cggccttgtg gatgcgctcg ttgcgataaa cgcccaggtc gtcgctcaag 21300
gtaagcacct tcagcgccat gcgcatgtag aacccctcga tctttacctc cttgtctatg 21360
ggaacgtaag gggtatggta tatcttgcgg gcgtaaaact tgcccaggct aagcatggaa 21420
tagttgatgg cggccacctt gtcagccagg ctcaagctgc gctcctgcac cactatgctc 21480
tgcaggatgt ttatcaaatc gagcagccag cggccctcgg gctctactat gtttagcagc 21540
gcatccctga atgcctcgtt gtccctgctg tgctgcacta taaggaacag ctgcgccatg 21600
agcggcttgc tatttgggtt ttgctccagc gcgcttacaa agtcccacag atgcatcagt 21660
cctatagcca cctcctcgcg cgccacaagc gtacgcacgt ggttgttaaa gcttttttga 21720
aagttaatct cctggttcac cgtctgctcg tatgcggtta ccaggtcggc ggccgccacg 21780
tgtgcgcgcg cgggactaat cccggttcgc gcgtcgggct caaagtcctc ctcgcgcagc 21840
aaccgctcgc gattcaggcc atgccgcagc tcgcgccctg cgtggaactt tcgatcccgc 21900
atctcctcgg gctcctctcc ctcgcggtcg cgaaacaggt tctgccgcgg cacgtacgcc 21960
tcacgcgtat cacgcttcag ctgcaccctt gggtaccgct caggagaggg cgctcctagc 22020
cgcgccaggc cctcgccctc ctccaagtcc aggtagtgcc gggcccggcg ccgcgggggt 22080
tcgtaatcac catctgctgc cgcgtcaacc gcggatgtcg cccctcctga cgcggtagga 22140
ggaggggagg gtgccctgca tgtctgccgc tgctcttgct cttgccgctg ctgaggaggg 22200
gggcgcatct gccgcagcac cggatgcatc tgggaaaagc aaaaaagggg ctcgtccctg 22260
tttccggagg aatttgcaag cggggtcttg catgacgggg aggcaaaccc ccgttcgccg 22320
cagtccggcc ggtccgagac tcgaaccggg ggtcccgcga ctcaaccctt ggaaaataac 22380
cctccggcta cagggagcga gccacttaat gctttcgctt tccagcctaa ccgcttacgc 22440
tgcgcgcggc cagtggccaa aaaagctagc gcagcagccg ccgcgcctgg aaggaagcca 22500

aaaggagcac tcccccgttg tctgacgtcg cacacctggg ttcgacacgc gggcggtaac 22560
cgcatggatc acggcggacg gccggatacg gggctcgaac cccggtcgtc cgccatgata 22620
cccttgcgaa tttatccacc agaccacgga agagtgcccg cttacaggct ctccttttgc 22680
acggtagagc gtcaacgatt gcgcgcgcct gaccggccag agcgtcccga ccatggagca 22740
ctttttgccg ctgcgcaaca tctggaaccg cgtccgcgac tttccgcgcg cctccaccac 22800
cgccgccggc atcacctgga tgtccaggta catctacgga tatcatcgcc ttatgttgga 22860
agatctcgcc cccggagccc cggccaccct acgctggccc ctctaccgcc agccgccgcc 22920
gcactttttg gtgggatacc agtacctggt gcggacttgc aacgactacg tatttgactc 22980
gagggcttac tcgcgtctca ggtacaccga gctctcgcag ccgggtcacc agaccgttaa 23040
ctggtccgtt atggccaact gcacttacac catcaacacg ggcgcatacc accgctttgt 23100
ggacatggat gacttccagt ctaccctcac gcaggtgcag caggccatat tagccgagcg 23160
cgttgtcgcc gacctagccc tgcttcagcc gatgaggggc ttcggggtca cacgcatggg 23220
aggaagaggg cgccacctac ggccaaactc cgccgccgcc gcagcgatag atgcaagaga 23280
tgcaggacaa gaggaaggag aagaagaagt gccggtagaa aggctcatgc aagactacta 23340
caaagacctg cgccgatgtc aaaacgaagc ctggggcatg gccgaccgcc tgcgcattca 23400
gcaggccgga cccaaggaca tggtgcttct gtcgaccatc cgccgtctca agaccgccta 23460
ctttaattac atcatcagca gcacctccgc cagaaacaac cccgaccgcc gcccgctgcc 23520
gcccgccacg gtgctcagcc taccttgcga ctgtgactgg ttagacgcct ttctcgagag 23580
gttttccgat ccggtcgatg cggactcgct caggtccctc ggcggcggag tacctacaca 23640
acaattgttg agatgcatcg ttagcgccgt atccctgccg catggcagcc ccccgccaac 23700
ccataaccgg gacatgacgg gcggcgtctt ccaactgcgc ccccgcgaga acggccgcgc 23760
cgtcaccgag accatgcgcc gtcgccgcgg ggagatgatc gagcgctttg tcgaccgcct 23820
cccggtgcgc cgtcgtcgcc gccgtgtccc ccctccccca ccgccgccag aagaagaaga 23880
aggggaggcc cttatggaag aggagattga agaagaagaa gaggcccctg tagcctttga 23940
gcgcgaggtg cgcgacactg tcgccgagct catccgtctt ctggaggagg agttaaccgt 24000
gtcggcgcgc aactcccagt ttttcaactt cgccgtggac ttctacgagg ccatggagcg 24060
ccttgaggcc ttgggggata tcaacgaatc cacgttgcga cgctgggtta tgtacttctt 24120
cgtggcagaa cacaccgcca ccaccctcaa ctacctcttt cagcgcctgc gaaactacgc 24180
cgtcttcgcc cggcacgtgg agctcaatct cgcgcaggtg gtcatgcgcg cccgcgatgc 24240
cgaagggggc gtggtctaca gccgcgtctg gaacgaggga ggcctcaacg ccttctcgca 24300
gctcatggcc cgcatttcca acgacctcgc cgccaccgtg gagcgagccg gacgcggaga 24360
tctccaggag gaagagatcg agcagttcat ggccgagatc gcctatcaag acaactcagg 24420
agacgtgcag gagattttgc gccaggccgc cgtcaacgac accgaaattg attctgtcga 24480
actctctttc aggctcaagc tcaccgggcc cgtcgtcttc acgcagaggc gccagattca 24540
ggagatcaac cgccgcgtcg tcgcgttcgc cagcaaccta cgcgcgcagc accagctcct 24600
gcccgcgcgc ggcgccgacg tgcccctgcc ccctctcccg gcgggtccgg agccccccct 24660
acctccgggg gctcgcccgc gtcaccgctt ttagatgcat catccaagga cacccccgcg 24720
gcccaccgcc cgccgcgcgg taccgtagtc gcgccgcggg gatgcggcct cttgcaagcc 24780
atcgacgccg ccaccaacca gcccctggaa attaggtatc acctggatct agcccgcgcc 24840
ctgacccgtc tatgcgaggt aaacctgcag gagctcccgc ctgacctgac gccgcgggag 24900
ctccagacca tggacagctc ccatctgcgc gatgttgtca tcaagctccg accgccgcgc 24960
gcggacatct ggactttggg ctcgcgcggc gtggtggtcc gatccaccgt aactcccctc 25020
gagcagccag acggtcaagg acaagcagcc gaagtagaag accaccagcc aaacccgcca 25080
ggcgaggggc tcaaattccc actctgcttc cttgtgcgcg gtcgtcaggt caacctcgtg 25140
caggatgtac agcccgtgca ccgctgccag tactgcgcac gtttttacaa aagccagcac 25200
gagtgttcgg cccgtcgcag ggacttctac tttcaccaca tcaatagcca ctcctccaat 25260
tggtggcggg agatccagtt cttcccgatc ggctcgcatc ctcgcaccga gcgtctcttt 25320
gtcacctacg atgtagagac ctatacttgg atgggggcct ttgggaagca gctcgtgccc 25380

ttcatgctgg tcatgaagtt cggcggagat gagcctctag tgactgccgc gcgagaccta 25440
gccgcgaacc ttggatggga ccgctgggaa caagacccgc ttaccttcta ctgcatcacc 25500
ccagaaaaaa tggccatagg tcgccagttt aggacctttc gcgaccacct gcaaatgcta 25560
atggcccgtg acctgtggag ctcattcgtc gcttccaacc ctcatcttgc agactgggcc 25620
ctttcagagc acgggctcag ctcccctgaa gagctcacct acgaggaact taaaaaattg 25680
ccttccatca agggcatccc gcgcttcttg gaactttaca ttgtgggcca caacatcaac 25740
ggctttgacg agatcgtgct cgccgcccag gtaattaaca accgttccga ggtgccggga 25800
cccttccgca tcacacgcaa ctttatgcct cgcgcgggaa agatactctt caacgatgtc 25860
accttcgccc tgccaaatcc gcgttccaaa aagcgcacgg actttttgct ctgggagcag 25920
ggcggatgcg acgacactga cttcaaatac cagtacctca aagtcatggt cagggacacc 25980
tttgcgctca cccacacctc gctccggaag gccgcgcagg catacgcgct acccgtagaa 26040
aagggatgct gcgcctacca ggccgtcaac cagttctaca tgctaggctc ttaccgttcg 26100
gaggccgacg ggtttccgat ccaagagtac tggaaagacc gcgaagagtt tgtcctcaac 26160
cgcgagctgt ggaaaaaaaa gggacaggat aagtatgaca tcatcaagga aaccctggac 26220
tactgcgccc tagacgtgca ggtcaccgcc gagctggtca acaagctgcg cgactcctac 26280
gcctccttcg tgcgtgacgc ggtaggtctc acagacgcca gcttcaacgt cttccagcgt 26340
ccaaccatat catccaactc acatgccatc ttcaggcaga tagtcttccg agcagagcag 26400
cccgcccgta gcaacctcgg tcccgacctc ctcgctccct cgcacgaact atacgattac 26460
gtgcgcgcca gcatccgcgg tggaagatgc taccctacat atcttggaat actcagagag 26520
cccctctacg tttacgacat ttgcggcatg tacgcctccg cgctcaccca ccccatgcca 26580
tggggtcccc cactcaaccc atacgagcgc gcgcttgccg cccgcgcatg gcagcaggcg 26640
ctagacttgc aaggatgcaa gatagactac ttcgacgcgc gcctgctgcc cggggtcttt 26700
accgtggacg cagacccccc ggacgagacg cagctagacc ccctaccgcc attctgctcg 26760
cgcaagggcg gccgcctctg ctggaccaac gagcgcctac gcggagaggt agccaccagc 26820
gttgaccttg tcaccctgca caaccgcggt tggcgcgtgc acctggtgcc cgacgagcgc 26880
accaccgtct ttcccgaatg gcggtgcgtt gcgcgcgaat acgtgcagct aaacatcgcg 26940
gccaaggagc gcgccgatcg cgacaaaaac caaaccctgc gctccatcgc caagttgctg 27000
tccaacgccc tctacgggtc gtttgccacc aagcttgaca acaaaaagat tgtcttttct 27060
gaccagatgg atgcggccac cctcaaaggc atcaccgcgg gccaggtgaa tatcaaatcc 27120
tcctcgtttt tggaaactga caatcttagc gcagaagtca tgcccgcttt tcagagggag 27180
tactcacccc aacagctggc cctcgcagac agcgatgcgg aagagagtga ggacgaacgc 27240
gcccccaccc ccttttatag ccccccttca ggaacacccg gtcacgtggc ctacacctac 27300
aaaccaatca ccttccttga tgccgaagag ggcgacatgt gtcttcacac cctggagcga 27360
gtggaccccc tagtggacaa cgaccgctac ccctcccact tagcctcctt cgtgctggcc 27420
tggacgcgag cctttgtctc agagtggtcc gagtttctat acgaggagga ccgcggaaca 27480
ccgctcgagg acaggcctct caagtctgta tacggggaca cggacagcct tttcgtcacc 27540
gagcgtggac accggctcat ggaaaccaga ggtaagaaac gcatcaaaaa gcatggggga 27600
aacctggttt ttgaccccga acggccagag ctcacctggc tcgtggaatg cgagaccgtc 27660
tgcggggcct gcggcgcgga tgcctactcc ccggaatcgg tatttctcgc gcccaagctc 27720
tacgccctca aaagtctgca ctgcccctcg tgcggcgcct cctccaaggg caagctgcgc 27780
gccaagggcc acgccgcgga ggggctggac tatgacacca tggtcaaatg ctacctggcc 27840
gacgcgcagg gcgaagaccg gcagcgcttc agcaccagca ggaccagcct caagcgcacc 27900
ctggccagcg cgcagcccgg agcgcacccc ttcaccgtga cccagactac gctgacgagg 27960
accctgcgcc cgtggaaaga catgaccctg gcccgtctgg acgagcaccg actactgccg 28020
tacagcgaaa gccgccccaa cccgcgaaac gaggagatat gctggatcga gatgccgtag 28080
agcaggtgac cgagctgtgg gaccgcctgg aactgcttgg tcaaacgctc aaaagcatgc 28140
ctacggcgga cggtctcaaa ccgttgaaaa actttgcttc cttgcaagaa ctgctatcgc 28200
tgggcggcga gcgccttctg gcggatttgg tcagggaaaa catgcgagtc agggacatgc 28260

ttaacgaagt ggcccccctg ctcagggatg acggcagctg cagctctctt aactaccagt 28320
tgcacccggt aataggtgtg atttacgggc ccaccggctg cggtaagtcg cagctgctca 28380
ggaacctgct ttcttcccag ctgatctccc ctaccccgga aaccgttttc ttcatcgccc 28440
cgcaggtaga catgatcccc ccatctgaac tcaaagcgtg ggaaatgcaa atctgtgagg 28500
gtaactacgc ccctgggccg gatggaacca ttataccgca gtctggcacc ctccgcccgc 28560
gctttgtaaa aatggcctat gacgatctca tcctggaaca caactatgac gttagtgatc 28620
ccagaaatat cttcgcccag gccgccgccc gtgggcccat tgccatcatt atggacgaat 28680
gcatggaaaa tcttggaggt cacaagggcg tctccaagtt cttccacgca tttccttcta 28740
agctacatga caaatttccc aagtgcaccg gatacactgt gctggtggtt ctgcacaaca 28800
tgaatccccg gagggatatg gctgggaaca tagccaacct aaaaatacag tccaagatgc 28860
atctcatatc cccacgtatg cacccatccc agcttaaccg ctttgtaaac acttacacca 28920
agggcctgcc cctggcaatc agcttgctac tgaaagacat ttttaggcac cacgcccagc 28980
gctcctgcta cgactggatc atctacaaca ccaccccgca gcatgaagct ctgcagtggt 29040
gctacctcca ccccagagac gggcttatgc ccatgtatct gaacatccag agtcaccttt 29100
accacgtcct ggaaaaaata cacaggaccc tcaacgaccg agaccgctgg tcccgggcct 29160
accgcgcgcg caaaacccct aaataaagac agcaagacac ttgcttgatc caaatccaaa 29220
cagagtctgg ttttttattt atgttttaaa ccgcattggg aggggaggaa gccttcaggg 29280
cagaaacctg ctggcgcaga tccaacagct gctgagaaac gacattaagt tcccgggtca 29340
aagaatccaa ttgtgccaaa agagccgtca acttgtcatc gcgggcggat gaacgggaag 29400
ctgcactgct tgcaagcggg ctcaggaaag caaagtcagt cacaatcccg cgggcggtgg 29460
ctgcagcggc tgaagcggcg gcggaggctg cagtctccaa cggcgttcca gacacggtct 29520
cgtaggtcaa ggtagtagag tttgcgggca ggacggggcg accatcaatg ctggagccca 29580
tcacattctg acgcaccccg gcccatgggg gcatgcgcgt tgtcaaatat gagctcacaa 29640
tgcttccatc aaacgagttg gtgctcatgg cggcggcggc tgctgcaaaa cagatacaaa 29700
actacataag acccccacct tatatattct ttcccaccct tannntaata gtaatcaatt 29760
acggggtcat tagttcatag cccatatatg gagttccgcg ttggtaaatg gcccgcctgg 29820
ctgaccgccc aacgaccccc gcccattgac gtcaataatg acgtatgttc ccatagtaac 29880
gccaataggg actttccatt gacgtcaatg ggtggagtat ttacggtaaa ctgcccactt 29940
ggcagtacat caagtgtatc atatgccaag tacgccccct attgacgtca atgacggtaa 30000
atggcccgcc tggcattatg cccagtacat gaccttatgg gactttccta cttggcagta 30060
catctacgta ttagtcatcg ctattaccat ggtgatgcgg ttttggcagt acatcaatgg 30120
gcgtggatag cggtttgact cacggggatt tccaagtctc caccccattg acgtcaatgg 30180
gagtttgttt tggcaccaaa atcaacggga ctttccaaaa tgtcgtaaca actccgcccc 30240
attgacgcaa atgggcggta ggcgtgtacg gtgggaggtc tatataagca gagctggttt 30300
agtgaaccgt cagatccgct agagatctgg taccaccatg gctgccatct ctacttccat 30360
ccctgtaatt tcacagcccc agttcacagc catgaatgaa ccacagtgct tctacaacga 30420
gtccattgcc ttcttttata accgaagtgg aaagcatctt gccacagaat ggaacacagt 30480
cagcaagctg gtgatgggac ttggaatcac tgtttgtatc ttcatcatgt tggccaacct 30540
attggtcatg gtggcaatct atgtcaaccg ccgcttccat tttcctattt attacctaat 30600
ggctaatctg gctgctgcag acttctttgc tgggttggcc tacttctatc tcatgttcaa 30660
cacaggaccc aatactcgga gactgactgt tagcacatgg ctccttcgtc agggcctcat 30720
tgacaccagc ctgacggcat ctgtggccaa cttactggct attgcaatcg agaggcacat 30780
tacggttttc cgcatgcagc tccacacacg gatgagcaac cggcgggtag tggtggtcat 30840
tgtggtcatc tggactatgg ccatcgttat gggtgctata cccagtgtgg gctggaactg 30900
tatctgtgat attgaaaatt gttccaacat ggcacccctc tacagtgact cttacttagt 30960
cttctgggcc attttcaact tggtgacctt tgtggtaatg gtggttctct atgctcacat 31020
ctttggctat gttcgccaga ggactatgag aatgtctcgg catagttctg gaccccggcg 31080
gaatcgggat accatgatga gtcttctgaa gactgtggtc attgtgcttg gggcctttat 31140

catctgctgg actcctggat tggttttgtt acttctagac gtgtgctgtc cacagtgcga 31200
cgtgctggcc tatgagaaat tcttccttct ccttgctgaa ttcaactctg ccatgaaccc 31260
catcatttac tcctaccgcg acaaagaaat gagcgccacc tttaggcaga tcctctgctg 31320
ccagcgcagt gagaacccca ccggccccac agaaggctca gaccgctcgg cttcctccct 31380
caaccacacc atcttggctg gagttcacag caatgatcac tctgtggttt atccctatga 31440
cgtccccgac tatgcctgac tcgagcctaa gcttctagat aagatatccg atcnntggag 31500
ttcgtgaccg ccgccgggat cactctcggc atggacgagc tgtacaagtc cggactcaga 31560
tccaccggat ctagataact gatcataatc agccatacca catttgtaga ggttttactt 31620
gctttaaaaa acctcccaca cctccccctg aacctgaaac ataaaatgaa tgcaattgtt 31680
gttgttaact tgtttattgc agcttataat ggttacaaat aaagcaatag catcacaaat 31740
ttcacaaata aagcattttt ttcactgcat tctagttgtg gtttgtccaa actcatcaat 31800
gtatcttaac gcgnnntaat agtaatcaat tacggggtca ttagttcata gcccatatat 31860
ggagttccgc gttacataac ttacggtaaa tggcccgcct ggctgaccgc ccaacgaccc 31920
ccgcccattg acgtcaataa tgacgtatgt tcccatagta acgccaatag ggactttcca 31980
ttgacgtcaa tgggtggagt atttacggta aactgcccac ttggcagtac atcaagtgta 32040
tcatatgcca agtacgcccc ctattgacgt caatgacggt aaatggcccg cctggcatta 32100
tgcccagtac atgaccttat gggactttcc tacttggcag tacatctacg tattagtcat 32160
cgctattacc atggtgatgc ggttttggca gtacatcaat gggcgtggat agcggtttga 32220
ctcacgggga tttccaagtc tccaccccat tgacgtcaat gggagtttgt tttggcacca 32280
aaatcaacgg gactttccaa aatgtcgtaa caactccgcc ccattgacgc aaatgggcgg 32340
taggcgtgta cggtgggagg tctatataag cagagctggt ttagtgaacc gtcagatccg 32400
ctagcgctac cggtcgccac catggtgagc aagggcgagg agctgttcac cggggtggtg 32460
cccatcctgg tcgagctgga cggcgacgta aacggccaca agttcagcgt gtccggcgag 32520
ggcgagggcg atgccaccta cggcaagctg accctgaagt tcatctgcac caccggcaag 32580
ctgcccgtgc cctggcccac cctcgtgacc accctgacct acggcgtgca gtgcttcagc 32640
cgctaccccg accacatgaa gcagcacgac ttcttcaagt ccgccatgcc cgaaggctac 32700
gtccaggagc gcaccatctt cttcaaggac gacggcaact acaagacccg cgccgaggtg 32760
aagttcgagg gcgacaccct ggtgaaccgc atcgagctga agggcatcga cttcaaggag 32820
gacggcaaca tcctggggca caagctggag tacaactaca acagccacaa cgtctatatc 32880
atggccgaca agcagaagaa cggcatcaag gtgaacttca agatccgcca caacatcgag 32940
gacggcagcg tgcagctcgc cgaccactac cagcagaaca cccccatcgg cgacggcccc 33000
gtgctgctgc ccgacaacca ctacctgagc acccagtccg ccctgagcaa agaccccaac 33060
gagaagcgcg atcacatggt cctgctggag ttcgtgaccg ccgccgggat cactctcggc 33120
atggacgagc tgtacaagtc cggactcaga tccaccggat ctagataact gatcataatc 33180
agccatacca catttgtaga ggttttactt gctttaaaaa acctcccaca cctccccctg 33240
aacctgaaac ataaaatgaa tgcaattgtt gttgttaact tgtttattgc agcttataat 33300
ggttacaaat aaagcaatag catcacaaat ttcacaaata aagcattttt ttcactgcat 33360
tctagttgtg gtttgtccaa actcatcaat gtatcttaac gcgnnnttac gcgctatgag 33420
taacacaaaa ttattcagat ttcacttcct cttattcagt tttcccgcga aaatggccaa 33480
atcttactcg gttacgccca aatttactac aacatccgcc taaaaccgcg cgaaaattgt 33540
cacttcctgt gtacaccggc gcacaccaaa aacgtcactt ttgccacatc cgtcgcttac 33600
atgtgttccg ccacacttgc aacatcacac ttccgccaca ctactacgtc acccgccccg 33660
ttcccacgcc cgcgccacgt cacaaactcc accccctcat tatcatattg gcttcaatcc 33720
aaaaggggca gagagctgga agggannntt aattaannnn nnnnnnnnnn nnnnnnnnnn 33780
nnnnnnnnnn nnncggcgca ttaagcgcgc gggtgtggtg gttacgcgca gcgtgaccgc 33840
tacacttgcc agcgccctag cgcccgctcc tttcgctttc ttcccttcct ttctcgccac 33900
gttcgccggc tttccccgtc aagctctaaa tcgggggctc cctttagggt tccgatttag 33960
agctttacgg cacctcgacc gcaaaaaact tgatttgggt gatggttcac gtagtgggcc 34020

atcgccctga tagacggttt ttcgcccttt gacgttggag tccacgttct ttaatagtgg 34080
actcttgttc caaactggaa caacactcaa ccctatcgcg gtctattctt ttgatttata 34140
agggatgttg ccgatttcgg cctattggtt aaaaaatgag ctgatttaac aaaaatttta 34200
acaaaattca gaagaactcg tcaagaaggc gatagaaggc gatgcgctgc gaatcgggag 34260
cggcgatacc gtaaagcacg aggaagcggt cagcccattc gccgccaagc tcttcagcaa 34320
tatcacgggt agccaacgct atgtcctgat agcggtccgc cacacccagc cggccacagt 34380
cgatgaatcc agaaaagcgg ccattttcca ccatgatatt cggcaagcag gcatcgccat 34440
gggtcacgac gagatcctcg ccgtcgggca tgctcgcctt gagcctggcg aacagttcgg 34500
ctggcgcgag cccctgatgc tcttcgtcca gatcatcctg atcgacaaga ccggcttcca 34560
tccgagtacg tgctcgctcg atgcgatgtt tcgcttggtg gtcgaatggg caggtagccg 34620
gatcaagcgt atgcagccgc cgcattgcat cagccatgat ggatactttc tcggcaggag 34680
caaggtgaga tgacaggaga tcctgccccg gcacttcgcc caatagcagc cagtcccttc 34740
ccgcttcagt gacaacgtcg agcacagctg cgcaaggaac gcccgtcgtg gccagccacg 34800
atagccgcgc tgcctcgtct tgcagttcat tcagggcacc ggacaggtcg gtcttgacaa 34860
aaagaaccgg gcgcccctgc gctgacagcc ggaacacggc ggcatcagag cagccgattg 34920
tctgttgtgc ccagtcatag ccgaatagcc tctccaccca agcggccgga gaacctgcgt 34980
gcaatccatc ttgttcaatc atgcgaaacg atcctcatcc tgtctcttga tcagagcttg 35040
atcccctgcg ccatcagatc cttggcggcg agaaagccat ccagtttact ttgcagggct 35100
tcccaacctt accagagggc gccccagctg gcaattccgg ttcgcttgct gtccataaaa 35160
ccgcccagtc tagctatcgc catgtaagcc cactgcaagc tacctgcttt ctctttgcgc 35220
ttgcgttttc ccttgtccag atagcccagt agctgacatt catccggggt cagcaccgtt 35280
tctgcggact ggctttctac gtgaaaagga tctaggtgaa gatccttttt nnnnnncaac 35340
aacgttgcgc aaactattaa ctggcgaact acttactcta gcttcccggc aacaattaat 35400
agactggatg gaggcggata aagttgcagg accacttctg cgctcggccc ttccggctgg 35460
ctggtttatt gctgataaat ctggagccgg tgagcgtggg tctcgcggta tcattgcagc 35520
actggggcca gatggtaagc cctcccgtat cgtagttatc tacacgacgg ggagtcaggc 35580
aactatggat gaacgaaata gacagatcgc tgagataggt gcctcactga ttaagcattg 35640
gtaactgtca gaccaagttt actcatatat actttagatt gatttaaaac ttcattttta 35700
atttaaaagg atctaggtga agatcctttt tgataatctc atgaccaaaa tcccttaacg 35760
tgagttttcg ttccactgag cgtcagaccc cgtagaaaag atcaaaggat cttcttgaga 35820
tccttttttt ctgcgcgtaa tctgctgctt gcaaacaaaa aaaccaccgc taccagcggt 35880
ggtttgtttg ccggatcaag agctaccaac tctttttccg aaggtaactg gcttcagcag 35940
agcgcagata ccaaatactg tccttctagt gtagccgtag ttaggccacc acttcaagaa 36000
ctctgtagca ccgcctacat acctcgctct gctaatcctg ttaccagtgg ctgctgccag 36060
tggcgataag tcgtgtctta ccgggttgga ctcaagacga tagttaccgg ataaggcgca 36120
gcggtcgggc tgaacggggg gttcgtgcac acagcccagc ttggagcgaa cgacctacac 36180
cgaactgaga tacctacagc gtgagcattg agaaagcgcc acgcttcccg aagggagaaa 36240
ggcggacagg tatccggtaa gcggcagggt cggaacagga gagcgcacga gggagcttcc 36300
agggggaaac gcctggtatc tttatagtcc tgtcgggttt cgccacctct gacttgagcg 36360
tcgatttttg tgatgctcgt caggggggcg gagcctatgg aaaaacgcca gcaacgcggc 36420
ctttttacgg ttcctggcct tttgctggcc ttttgctcac atgttctttc ctgcgttatc 36480
ccctgattct gtggataacc gtattaccgc ctttgagtga gctgataccg ctcgccgcag 36540
ccgaacgacc gagcgcagcg agtcagtgag cgaggaagcg gaagagcgcc tgatgcggta 36600
ttttctcctt acgcatctgt gcggtatttc acaccgcata tggtgcactc tcagtacaat 36660
ctgctctgat gccgcatagt taagccagta tacactccgc tatcgctacg tgactgggtc 36720
atggctgcgc cccgacaccc gccaacaccc gctgacgcgc cctgacgggc ttgtctgctc 36780
ccggcatccg cttacagaca agctgtgacc gtctccggga gctgcatgtg tcagaggttt 36840
tcaccgtcat caccgaaacg cgcgaggcag ctgcggtaaa gctcatcagc gtggtcgtga 36900

agcgattcac agatgtctgc ctgttcatcc gcgtccagct cgttgagttt ctccagaagc 36960
gttaatgtct ggcttctgat aaagcgggcc atgttaaggg cggttttttc ctgtttggtc 37020
acttgatgcc tccgtgtaag ggggaatttc tgttcatggg ggtaatgata ccgatgaaac 37080
gagagaggat gctcacgata cgggttactg atgatgaaca tgcccggtta ctggaacgtt 37140
gtgagggtaa acaactggcg gtatggatgc ggcgggacca gagaaaaatc actcagggtc 37200
aatgccagcg cttcgttaat acagatgtag gtgttccaca gggtagccag cagcatcctg 37260
cgatgcagat ccggaacata atggtgcagg gcgctgactt ccgcgtttcc agactttacg 37320
aaacacggaa accgaagacc attcatgttg ttgctcaggt cgcagacgtt ttgcagcagc 37380
agtcgcttca cgttcgctcg cgtatcggtg attcattctg ctaaccagta aggcaacccc 37440
gccagcctag ccgggtcctc aacgacagga gcacgatcat gcgcacccgt ggccaggacc 37500
caacgctgcc cgagatgcgc cgcgtgcggc tgctggagat ggcggacgcg atggatatgt 37560
tctgccaagg gttggtttgc gcattcacag ttctccgcaa gaattgattg gctccaattc 37620
ttggagtggt gaatccgtta gcgaggtgcc gccggcttcc attcaggtcg aggtggcccg 37680
gctccatgca ccgcgacgca acgcggggag gcagacaagg tatagggcgg cgcctacaat 37740
ccatgccaac ccgttccatg tgctcgccga ggcggcataa atcgccgtga cgatcagcgg 37800
tccagtgatc gaagttaggc tggtaagagc cgcgagcgat ccttgaagct gtccctgatg 37860
gtcgtcatct acctgcctgg acagcatggc ctgcaacgcg ggcatcccga tgccgccgga 37920
agcgagaaga atcataatgg ggaaggccat ccagcctcgc gtcgcgaacg ccagcaagac 37980
gtagcccagc gcgtcggccg ccatgccggc gataatggcc tgcttctcgc cgaaacgttt 38040
ggtggcggga ccagtgacga aggcttgagc gagggcgtgc aagattccga ataccgcaag 38100
cgacaggccg atcatcgtcg cgctccagcg aaagcggtcc tcgccgaaaa tgacccagag 38160
cgctgccggc acctgtccta cgagttgcat gataaagaag acagtcataa gtgcggcgac 38220
gatagtcatg ccccgcgccc accggaagga gctgactggg ttgaaggctc tcaagggcat 38280
cggtcgagga caggnnngga tcctta 38306

<210> 6
<211> 40
<212> DNA
<213> Homo sapiens

<400> 6
gcggggggta ccaccatggc tgccatctct acttccatcc 40

<210> 7
<211> 60
<212> DNA
<213> Homo sapiens

<400> 7
gcggggctcg agtcacttgt cgtcgtcgtc cttatagtca accacagagt gatcattgct 60

<210> 8
<211> 1095
<212> DNA
<213> Homo sapiens

<400> 8
atggctgcca tctctacttc catccctgta atttcacagc cccagttcac agccatgaat 60
gaaccacagt gcttctacaa cgagtccatt gccttctttt ataaccgaag tggaaagcat 120
cttgccacag aatggaacac agtcagcaag ctggtgatgg gacttggaat cactgtttgt 180
atcttcatca tgttggccaa cctattggtc atggtggcaa tctatgtcaa ccgccgcttc 240
cattttccta tttattacct aatggctaat ctggctgctg cagacttctt tgctgggttg 300
gcctacttct atctcatgtt caacacagga cccaatactc ggagactgac tgtcagcaca 360
tggctccttc gtcagggcct cattgacacc agcctgacgg catctgtggc caacttactg 420
gctattgcaa tcgagaggca cattacggtt ttccgcatgc agctccacac acggatgagc 480
aaccggcggg tagtggtggt cattgtggtc atctggacta tggccatcgt tatgggtgct 540
atacccagtg tgggctggaa ctgtatctgt gatattgaaa attgttccaa catggcaccc 600
ctctacagtg actcttactt agtcttctgg gccattttca acttggtgac ctttgtggta 660
atggtggttc tctatgctca catctttggc tatgttcgcc agaggactat gagaatgtct 720
cggcatagtt ctggaccccg gcggaatcgg gataccatga tgagtcttct gaagactgtg 780
gtcattgtgc ttggggcctt tatcatctgc tggactcctg gattggtttt gttacttcta 840
gacgtgtgct gtccacagtg cgacgtgctg gcctatgaga aattcttcct tctccttgct 900
gaattcaact ctgccatgaa ccccatcatt tactcctacc gcgacaaaga aatgagcgcc 960
acctttaggc agatcctctg ctgccagcgc agtgagaacc ccaccggccc cacagaaggc 1020
tcagaccgct cggcttcctc cctcaaccac accatcttgg ctggagttca cagcaatgac 1080
cactctgtgg tttag 1095

## Claims

1. Myocardial cell of a mammal which cell contains an adenoviral vector sequence for simultaneous expression of G protein coupled receptor EDG2 and GFP.

2. Myocardial cell of a mammal as claimed in claim 1 wherein the adenoviral vector sequence consists of a recombinant E1/E3 deficient adenovirus which expresses the G protein coupled receptor EDG2 and GFP under control of two independent promoters.

3. Myocardial cell of a mammal as claimed in claim 2 wherein the two independent promoters are two CMV promoters.

4. Myocardial cell of a mammal as claimed in one of claims 1 to 3 which contains protein of G protein coupled receptor EDG2 and protein of GFP.

5. Myocardial cell of a mammal as claimed in one of claims 1 to 4 wherein the mammal is a rabbit, mouse or rat.

6. Production of a myocardial cell as claimed in claims 1 to 5 wherein
a] the heart of a mammal is removed by state of a veterinary medicine operative techniques,
b] the heart is perfused and digested with collagenase,
c] the isolated cardiomyocytes are infected with an adenoviral vector consisting of a recombinant E1/E3 deficient adenovirus which expresses the G protein coupled receptor EDG2 and GFP under control of two independent promoters.

7. Mammal having a myocardium which contains an adenoviral vector for simultaneous expression of a G protein coupled receptor EDG2 and GFP.

8. Mammal as claimed in claim 7 wherein the adenoviral vector sequence consists of a recombinant E1/E3 deficient adenovirus which expresses the G protein coupled receptor EDG2 and GFP under control of two independent promoters.

9. Mammal as claimed in claim 8 wherein the two independent promoters are two CMV promoters.

10. Mammal as claimed in one of claims 7 to 9 which myocardium contains protein of G protein coupled receptor EDG2 and protein of GFP.

11. Mammal as claimed in one of claims 7 to 10 being a rabbit, a mouse, or a rat.

12. Production of a mammal as claimed in claims 7 to 11 wherein
a] an adenoviral vector sequence for simultaneous expression of G protein coupled receptor EDG2 and GFP is provided,
b] a mammal is provided,
c] the adenoviral vector sequence from a] is transferred into the myocardium of the mammal from b] by means of a catheter.

13. Use of a mammal of claims for producing a myocardial cell as claimed in claims 1 to 6 for performing of claims 14 and 15.

14. Method for identification of a compound, which modifies the activity of G protein coupled receptor EDG2, wherein
a] a transformed cell from a heart muscle which expresses the receptor EDG2 or a fusion protein comprising the receptor EDG2 is provided,
b] possibly a treatment of the cell from a] is performed by use of isoproterenol and/or lysophosphatidic acid,
c] a chemical compound is provided,
d] the cell from a] or b] is brought in contact with the chemical compound from c],
e] the contractility of a cell from d] is determined and is brought in relation to contractility of a cell which has the same characteristics as a cell from a] but has not brought in contact with a chemical compound from c] wherein a relative enhancement or reduction of contractility of the cell which has brought in contact with a chemical compound according to d] by this compound demonstrates the ability of such compound to modify the activity of receptor EDG2.

15. Method for identification of a compound which modifies the activity of G protein coupled receptor EDG2, wherein
a] a transformed cell from a heart muscle which expresses the receptor EDG2 or a fusion protein comprising the receptor EDG2 is provided,
b] possibly a treatment of the cell from a] is performed by use of isoproterenol and/or lysophosphatidic acid,
c] a chemical compound is provided,
d] the cell from a] or b] us brought in contact with the chemical compound from c],
e] the contractility of a cell from d] is determined and is brought in relation to contractility of a cell of same cell type as a cell according to a] but which does not express a receptor EDG2 or a fusion protein comprising a receptor EDG2 wherein a relative enhancement or reduction of contractility of the cell which expresses a receptor EDG2 or a fusion protein comprising a receptor EDG2 by a compound demonstrates the ability of such compound to modify the activity of receptor EDG2.

16. Recombinant adenoviral vector consisting of one polynucleotide of the following groups:
a] a polynucleotide having a sequence as specified in SEQ ID NO. 5,
b] a polynucleotide, which is 95 % identical to the polynucleotide of SEQ ID NO. 5,
c] a polynucleotide, which is at least of the same length as the polynucleotide of SEQ ID NO. 5 and which hybridizes to a polynucleotide of SEQ ID NO. 5 when applying highly stringent hybridization conditions.

17. Recombinant adenoviral vector as claimed in claim 16 comprising a polynucleotide sequence which is encoding a protein of SEQ ID NO. 2.

18. Use of an adenoviral vector of claims 16 and 17 for constructing of transgenic mammals wherein the G protein coupled receptor EDG2 is transiently or permanently expressed in at least one tissue.

19. Use of an adenoviral vector of claim 18, wherein the tissue is part of the heart.
